# EUROPÄISCHE PATENTSCHRIFT

(11) **EP 2 506 808 B1**
(45) Veröffentlichungstag und Bekanntmachung des Hinweises auf die Patenterteilung: **22.07.2015**
(21) Anmeldenummer: 10790352.8
(22) Anmeldetag: 01.12.2010
(51) Int. Cl.: A61F 2/90

(54) **MEDIZINISCHE VORRICHTUNG ZUR EINFUHR IN EIN HOHLORGAN UND VERFAHREN ZUR HERSTELLUNG EINER SOLCHEN VORRICHTUNG**
MEDICAL DEVICE FOR INSERTION INTO A HOLLOW ORGAN AND METHOD FOR PRODUCING SUCH A DEVICE
DISPOSITIF MÉDICAL À INTRODUIRE DANS UN ORGANE CREUX ET PROCÉDÉ POUR LA FABRICATION D'UN TEL DISPOSITIF

(30) Priorität: 01.12.2009 DE 102009056450
(43) Veröffentlichungstag der Anmeldung: 10.10.2012
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Kilchert, Jochen
(86) Internationale Anmeldenummer: PCT/EP2010/007302
(87) Internationale Veröffentlichungsnummer: WO 2011/066960

(56) Entgegenhaltungen:
- WO-A1-2010/049123
- DE-A1- 10 127 602

## Beschreibung

Die Erfindung bezieht sich auf eine medizinische Vorrichtung zur Einfuhr in ein Hohlorgan sowie auf ein Verfahren zur Herstellung einer derartigen Vorrichtung. Eine gattungsgemäße Vorrichtung mit den Merkmalen des Oberbegriffs des Anspruchs 1 ist beispielsweise aus DE 101 27 602 A1 bekannt.

Für die Behandlung von Aneurysmen, von Stenosen und für die Aufweitung von Thromben sind Gitterstrukturen geeignet, die eine erhöhte Feinmaschigkeit aufweisen. Durch die Feinmaschigkeit können die Strömungsverhältnisse im Aneurysma beeinflusst werden, insbesondere durch eine Verlangsamung der Strömung im Aneurysma mit dem Ziel, die Gerinnung und Verödung des Aneurysmas zu bewirken. Die feinmaschige Gitterstruktur fördert außerdem das schnelle Wachstum von Zellen (Endothelialisierung). Bei der Ausweitung von Stenosen und von Thromben werden überdies Partikel durch die Feinmaschigkeit gut blockiert.

Eine erhöhte Feinmaschigkeit wird insbesondere durch Geflechte erzielt, die sich daher für diese Art des Einsatzes besonders gut eignen.

Geflechte werden außerdem im endovaskulären Bereich immer dann gebraucht, wenn eine erhöhte Flexibilität verlangt wird. Beispielsweise können Körbe zum Schutz vor distaler Embolie oder zur Entfernung von Thromben in der Form von Geflechten hergestellt werden. Durch die gute Flexibilität von Geflechten ist die Behandlung auch an stark gewundenen Stellen, wie beispielsweise im Hirnbereich, möglich. Körbe können auch in anderen Bereichen eingesetzt werden, wie beispielsweise in der Blase zur Entfernung von Steinen. Die Flexibilität ist auch im Zusammenhang mit der Behandlung von Aneurysmen oder Stenosen von Vorteil, wenn sich diese an stark gewundenen Gefäßstellen befinden.

Es ist bekannt, Geflechte mit einer offenen Struktur herzustellen. Dies bedeutet, dass die Drähte des Geflechtes freie Enden aufweisen. Dadurch können die Drähte als langer Strang geflochten werden, der dann auf die geeignete Länge geschnitten wird. Dadurch ergeben sich verschiedene Einheiten mit jeweils offenen bzw. freien Drahtenden. Diese Herstellungstechnik ist dann geeignet, wenn das Geflecht viele Drähte aufweist und die Einzelanfertigung einzelner Einheiten unwirtschaftlich ist. Geflechte mit einer großen Anzahl von Drähten sind sehr feinmaschig und weisen die vorstehend genannten Vorteile auf.

Alternativ können Geflechte mit geschlossenen Schlaufen hergestellt werden. Dies erfordert eine Einzelherstellung jedes Geflechtes. Die so hergestellten Geflechte haben den Vorteil, dass die durch die Schlaufen abgerundeten Geflechtenden atraumatisch wirken und das Risiko einer Verletzung der Gefäßwand senken.

Ein Beispiel eines Geflechts mit geschlossenen Schlaufen ist in der eingangs genannten DE 101 27 602 A1 offenbart. Die Druckschrift beschreibt einen Stent zur Implantation im menschlichen Körper mit einem hohlzylindrischen Körper, der aus einem Geflecht hergestellt ist. An den Geflechtenden sind die freien Enden der Drähte des Geflechts zusammengeführt und verbunden derart, dass an den Geflechtenden Schlaufen bzw. Maschen gebildet sind. Die Schlaufen an den Geflechtenden führen zu einer Abschlusskante des Geflechts mit einer gezackten Kontur.

Sowohl Geflechte, bei denen die Drähte frei enden als auch Geflechte mit geschlossenen Schlaufen an den Geflechtenden, wie beispielsweise in DE 101 27 602 A1 beschrieben, haben den Nachteil, dass die freien Drahtenden bzw. die Schlaufen ein Wiedereinziehen des Geflechts in einen Katheter behindern oder sogar völlig verhindern, nachdem dieses aus dem Katheter in das Gefäß entlassen wurde. Bei Geflechten mit freien Drahtenden am Geflechtende besteht überdies im Hinblick auf die Wiedereinziehbarkeit des Geflechts das Problem, dass ein Führungsdraht zum Wiedereinziehen mit dem Geflecht praktisch nicht verbunden werden kann. Selbst wenn das Geflecht an einer Stelle mit einem Führungsdraht verbunden wäre, der das Geflecht in den Katheter einzieht, würden sich die freien Drähte an der Katheteröffnung verkanten. Dabei würde es zu einer Beschädigung des Geflechts und des Katheters kommen. Eine Zurückziehen des Geflechts ist daher nicht möglich.

Auch ein Geflecht, das mit geschlossenen Schlaufen endet, lässt sich nicht in den Katheter wieder einziehen, nachdem dieses vollständig entlassen wurde. Ein solches Geflecht weist mehrere Zipfel am Geflechtende auf, die sich beim Einziehen in den Katheter verkanten. Dies ist insbesondere dann der Fall, wenn das Geflecht an einer einzigen Stelle mit einem Führungsdraht verbunden ist.

Die nachveröffentlichte WO 2010/049123 A1 offenbart beispielsweise einen Stent mit einem Geflecht aus mehreren Drähten, wobei jeweils wenigstens zwei Drähte zu einem Geflechtende verbunden sind. Dadurch sind auf dem Umfang des Geflechts mehrere Endmaschen gebildet, die das Geflecht axial begrenzen. Somit weist der bekannte Stent an seinem Geflechtende einen gezackten bzw. diskontinuierlichen Verlauf auf. Die einzelnen Endmaschen können sich bei dem Versuch, den Stent in einen Katheter zurückzuziehen, an der Katheterspitze verkanten. Die WO 2010/049123 A1 offenbart eine medizinische Vorrichtung gemäß dem Oberbegriff von Anspruch 1.

Der Erfindung liegt die Aufgabe zugrunde, eine medizinische Vorrichtung zur Einfuhr in ein Hohlorgan anzugeben, die ein Geflecht umfasst und dessen axiales Geflechtende so ausgebildet ist, dass die medizinische Vorrichtung in ein Zufuhrsystem, beispielsweise in einen Katheter, wieder eingezogen werden kann, selbst wenn die medizinische Vorrichtung vollständig oder zumindest fast vollständig aus dem Zufuhrsystem entlassen wurde. Der Erfindung liegt ferner die Aufgabe zugrunde, ein Verfahren zur Herstellung einer derartigen Vorrichtung anzugeben.

Erfindungsgemäß wird die Aufgabe im Hinblick auf die Vorrichtung durch den Gegenstand des Anspruchs 1 und im Hinblick auf das Verfahren durch den Gegenstand des Anspruchs 15 gelöst.

Die Erfindung beruht auf dem Gedanken, eine medizinische Vorrichtung zur Einfuhr in ein Hohlorgan mit einem Hohlkörper anzugeben, der ein Geflecht aus Drahtelementen mit einer Reihe von Endmaschen aufweist, die ein axiales Geflechtende begrenzen. Die Endmaschen umfassen äußere Drahtelemente, die eine Abschlusskante des Geflechts bilden und in innere Drahtelemente übergehen, die innerhalb des Geflechts angeordnet sind.

Ein erster Abschnitt der Abschlusskante und ein zweiter Abschnitt der Abschlusskante weisen jeweils mehrere äußere Drahtelemente auf, die zusammen einen umlaufenden Rand der Abschlusskante bilden. Der Rand ist angepasst derart, dass das axiale Geflechtende des Hohlkörpers in ein Zufuhrsystem einziehbar ist.

Die äußeren Drahtelemente des ersten Abschnitts sind zur Bildung der Abschlusskante entlang dieser einander unmittelbar nachgeordnet. Die äußeren Drahtelemente weisen jeweils eine erste Axialkomponente auf, die in Längsrichtung L des Hohlkörpers verläuft.

Die äußeren Drahtelemente des zweiten Abschnitts sind zur Bildung der Abschlusskante entlang dieser einander unmittelbar nachgeordnet. Die äußeren Drahtelemente weisen jeweils eine zweite Axialkomponente auf, die in Längsrichtung L des Hohlkörpers verläuft. Die zweite Axialkomponente ist der ersten Axialkomponente entgegengesetzt, wobei die beiden Axialkomponenten auf dieselbe Umlaufrichtung des Randes bezogen sind.

Der Hohlkörper hat allgemein eine längliche Form mit einer Längsrichtung und ist dazu geeignet, durch ein Zufuhrsystem, beispielsweise durch einen Katheter in ein Hohlorgan, insbesondere in ein menschliches Hohlorgan eingeführt zu werden. Dabei entspricht die Längsrichtung des Hohlkörpers derjenigen Richtung, in der der Hohlkörper durch das Zufuhrsystem bewegt und im Hohlorgan freigesetzt wird. Der Begriff Längsrichtung des Hohlkörpers entspricht somit dem Begriff Bewegungsrichtung des Hohlkörpers im Zufuhrsystem. Die Bewegungsrichtung des Hohlkörpers im Zufuhrsystem kann die Vorschubbewegung umfassen beim Freisetzen des Hohlkörpers ebenso wie die Rückzugsbewegung beim Wiedereinziehen des Hohlkörpers in das Zufuhrsystem.

Unter dem Begriff Endmaschen werden die äußeren Maschen des Geflechts verstanden, die ein Geflechtende begrenzen. Bei einem länglichen Hohlkörper handelt es sich bei dem Geflechtende um ein axiales Geflechtende. Die äußeren Drahtelemente der Endmaschen sind die auf der Außenseite der Endmaschen angeordneten Drahtelemente, die die Begrenzung des Geflechtendes darstellen. Die äußeren Drahtelemente bilden dabei eine Abschlusskante des Geflechts und gehen in innere Drahtelemente über, die innerhalb des Geflechts angeordnet sind.

Die äußeren Drahtelemente können somit auch als kantenbildende Drahtelemente und die inneren Drahtelemente als geflechtbildende Drahtelemente bezeichnet werden.

Der erste Abschnitt der Abschlusskante und der zweite Abschnitt der Abschlusskante bilden jeweils eine Teilkante, die das Geflecht in Längsrichtung des Hohlkörpers begrenzt. Jeder Abschnitt weist mehrere äußere Drahtelemente auf bzw. ist aus mehreren äußeren Drahtelementen gebildet, die zusammen einen umlaufenden Rand der Abschlusskante bilden. Der umlaufende Rand ist derart angepasst, dass das axiale Geflechtende des Hohlkörpers in ein Zufuhrsystem einziehbar ist. Dies bedeutet, dass das Hohlorgan in Längsrichtung mit dem umlaufenden Rand in das Zufuhrsystem rückholbar ist, nachdem der Hohlkörper vollständig oder zumindest überwiegend aus dem Zufuhrsystem entlassen wurde.

Die äußeren Drahtelemente der beiden Abschnitte sind zur Bildung der Abschlusskante entlang dieser jeweils einander unmittelbar nachgeordnet. Dies bedeutet, dass die äußeren Drahtelemente des ersten Abschnitts eine erste Einheit des umlaufenden Randes bilden, die sich dadurch auszeichnet, dass die äußeren Drahtelemente dieser ersten Einheit jeweils eine erste Axialkomponente aufweisen, die in Längsrichtung des Hohlkörpers verläuft.

Dasselbe gilt in analoger Weise für die äußeren Drahtelemente des zweiten Abschnitts, die zur Bildung der Abschlusskante entlang dieser einander unmittelbar nachgeordnet sind und eine zweite Einheit des umlaufenden Randes bilden. Die äußeren Drahtelemente des zweiten Abschnitts zeichnen sich dadurch aus, dass diese jeweils eine zweite Axialkomponente aufweisen, die in Längsrichtung des Hohlkörpers verläuft. Die beiden Axialkomponenten sind in entgegengesetzter Richtung angeordnet, wobei die beiden Axialkomponenten auf dieselbe Umlaufrichtung des Randes bezogen sind. Dies bedeutet, dass eine Axialkomponente in Längsrichtung des Hohlkörpers nach vorne und die andere Axialkomponente in Längsrichtung des Hohlkörpers nach hinten weist. Mit anderen Worten weist eine der beiden Axialkomponenten in proximale Richtung, d.h. in Richtung des Anwenders und die andere der beiden Axialkomponenten in distale Richtung, d.h. vom Anwender bzw. Arzt weg. Die unterschiedliche Richtung der Axialkomponenten wird dadurch ausgedrückt, dass diese auf dieselbe Umlaufrichtung des Randes bezogen sind. Unter derselben Umlaufrichtung des Randes wird beispielsweise die Bewegung eines imaginären Punktes auf dem Rand in ein und derselben Richtung verstanden. Dabei kommt es nicht darauf an, ob die Bewegung dieses imaginären Punktes im Uhrzeigersinn oder im Gegenuhrzeigersinn auf dem Rand verläuft. Für die Definition der Richtung der Axialkomponenten genügt es, wenn die Umlaufrichtung bzw. der imaginäre Umlauf im gleichen Sinn erfolgt.

Durch die neue Flechtart ist das Geflecht so aufgebaut, dass die Endkontur des Geflechts, d.h. die Kontur im Bereich der Abschlusskante zumindest an einem axialen Geflechtende keine überstehenden Kanten, bzw. keine entgegen der Einzugsrichtung des Geflechtendes in das Zufuhrsystem vorstehenden Kanten aufweist. Vielmehr wird durch den umlaufenden Rand der Abschlusskante und die erfindungsgemäß orientierten äußeren Drahtelemente erreicht, dass das Geflechtende in das Zufuhrsystem eingezogen werden kann, ohne dass Teile des Geflechtendes radial nach außen über die Öffnung des Zufuhrsystems vorstehen und das Wiedereinziehen behindern. Die äußeren Drahtelemente bilden eine gemeinsame kompakte Abschlusskante.

Die Funktion der Einziehbarkeit in ein Zufuhrsystem, insbesondere in einen Katheter, nachdem die medizinische Vorrichtung vollständig entlassen wurde kann, sowohl bei Implantaten als auch bei medizinischen Geräten eingesetzt werden, die nur temporär in einem Gefäß freigesetzt werden, wie beispielsweise bei Körben, insbesondere Thrombosefänger. Bei Implantaten besteht der Vorteil darin, dass die Positionierung des Systems, beispielsweise die Positionierung von Stents, nach deren Entlassung korrigiert werden kann. Das Implantat kann dabei vollständig im Gefäß entlassen werden und sich an die Gefäßwand anlegen. Nach Überprüfung der Position des Implantats kann der Anwender, wenn eine falsche Positionierung festgestellt wird, das System wieder einziehen und dieses neu, beispielsweise vor einem Aneurysma oder einer Stenose positionieren. Bei temporär freigesetzten Vorrichtungen, wie beispielsweise bei Körben oder Filtern dient die Funktion der Wiedereinziehbarkeit dazu, die im Korb bzw. Filter gefangenen Partikel, beispielsweise Gerinnsel aus dem Gefäß oder aus anderen Hohlorganen zu entfernen.

Die Erfindung hat ferner den Vorteil, dass das Geflecht an einer einzigen Stelle des Geflechtendes mit einem Betätigungselement zum Zurückziehen, beispielsweise mit einem Führungsdraht verbunden werden kann, um die Funktion der Wiedereinziehbarkeit zu realisieren. Auf Grund der Erfindung ist es nicht erforderlich, auch wenn dies nicht ausgeschlossen ist, dass die einzelnen Maschen mit dem Führungsdraht verbunden werde, um so eine Orientierung der Endmaschen radial nach innen zu erreichen, wodurch die Funktion der Wiedereinziehbarkeit unterstützt würde. Um die Endmaschen nach innen zu orientieren, wären Verbindungsdrähte zwischen den Endmaschen und dem Führungsdraht erforderlich, was dazu führen würde, dass das proximale Ende des Korbes durch die Verbindungsdrähte versperrt wäre. Die Verbindungsdrähte würden beispielsweise das Einfangen eines Thrombus behindern. Durch die Erfindung wird hingegen die Voraussetzung geschaffen, eine Geflechtkonfiguration mit einem offenen Geflechtlumen, insbesondere bei Implantaten, wie Stents , bzw. mit einem offenen proximalen Geflechtende, wie bspw. bei Körben, deren distales Ende geschlossen ist, nach vollständiger Freisetzung in das Zufuhrsystem wiedereinzuziehen. Die Erfindung ist auch auf Geflechtkonfigurationen mit einem geschlossenen Geflechtlumen anwendbar.

Im Hinblick auf das erfindungsgemäße Verfahren beruht die Erfindung darauf, ein Verfahren zur Herstellung einer medizinischen Vorrichtung anzugeben, bei dem ein Hohlkörper aus Drahtelementen geflochten und eine erste Reihe von nebeneinander angeordneten Endmaschen gebildet wird, die ein axiales Geflechtende begrenzen. Dabei umfasst jede Endmasche wenigstens ein äußeres Drahtelement. Beim Flechten werden die äußeren Drahtelemente der Endmaschen so angeordnet, dass diese zusammen eine Abschlusskante des Geflechtendes bilden, wobei im Geflecht angeordnete innere Drahtelemente zur Bildung der äußeren Drahtelemente umgelenkt werden. Die Umlenkung erfolgt derart, dass die Windungsrichtung und/oder die Richtung der Axialkomponente bezogen auf die Längsachse der inneren Drahtelemente geändert wird. Die Änderung der Windungsrichtung bzw. die Änderung der Richtung der Axialkomponente bezogen auf die Längsachse L erfolgt beim Übergang der inneren auf die äußeren Drahtelemente.

Bei einer bevorzugten Ausführungsform der Erfindung weisen die äußeren Drahtelemente des ersten Abschnitts eine erste Umfangskomponente und die äußeren Drahtelemente des zweiten Abschnitts eine zweite Umfangskomponente auf, wobei die erste und zweite Umfangskomponente in derselben Umfangsrichtung des Hohlkörpers verlaufen. Die Umfangsrichtung des Hohlkörpers ist generell die Richtung, in der sich die Wandung des Hohlkörpers erstreckt. Beispielsweise ist bei einem zylindrischen Hohlkörper die Umfangsrichtung diejenige Richtung, in der sich die Mantelfläche des Hohlkörpers erstreckt. Im Unterschied zur Umfangsrichtung, die allgemein auf die Wandung des Hohlkörpers bezogen ist, wird unter der Umlaufrichtung des Randes die sich aus der Kontur des Randes ergebende Erstreckung verstanden. Beispielsweise entspricht die Umlaufrichtung des Randes der Umfangsrichtung eines hohlzylindrischen Körpers, wenn sich der Rand in einer Ebene erstreckt, die senkrecht zur Längsachse des Hohlkörpers verläuft. Wenn beispielsweise die Ebene, in der der Rand liegt, geneigt bezogen auf die Längsachse ist, sind die Umlaufrichtung des Randes und die Umfangsrichtung des Hohlkörpers unterschiedlich.

Bei einer weiteren bevorzugten Ausführungsform der Erfindung ist die Abschlusskante bezogen auf die Längsachse des Hohlkörpers schräg angeordnet. Dadurch wird das Wiedereinziehen des Hohlkörpers bzw. der medizinischen Vorrichtung in ein Zufuhrsystem weiter verbessert, da durch die schräge Abschlusskante eine in Längsrichtung proximale Spitze des Geflechtendes gebildet wird, die beim Wiedereinziehen zuerst in das Zufuhrsystem hineinbewegt wird und eine Führungsfunktion erfüllt.

Bei einer besonders bevorzugten Ausführungsform der Erfindung erstreckt sich wenigstens ein erstes äußeres Drahtelement einer Endmasche in den Bereich einer anderen Endmasche, wobei sich das erste äußere Drahtelement und wenigstens ein zweites äußeres Drahtelement der anderen Endmasche überlappen und zusammen entlang der Abschlusskante angeordnet sind. Die Überlappung der beiden Drahtelemente bedeutet, dass diese im Bereich der anderen Endmasche zusammen die Abschlusskante bilden und zusammen entlang dieser angeordnet sind. Diese Anordnung der äußeren Drahtelemente bildet eine bevorzugte Möglichkeit, wie die Ausbildung eines einziehbaren umlaufenden Randes bzw. einer solchen Abschlusskante realisiert werden kann. Die Überlappung der Drahtelemente erfolgt dabei hauptsächlich im ersten Abschnitt bzw. im zweiten Abschnitt der Abschlusskante.

Vorzugsweise sind das wenigstens eine erste äußere Drahtelement und das wenigstens eine zweite äußere Drahtelement verbunden. Bei den zusammen entlang der Abschlusskante angeordneten und sich überlappenden Drahtelementen hat die Verbindung der Drahtelemente den Vorteil, dass das Geflechtende, insbesondere die Abschlusskante stabil ist. Dadurch wird die Funktion der Einziehbarkeit weiter verbessert. Ferner wird ein besonders glatter umlaufender Rand gebildet, der sich nicht nur gut einziehen lässt, sondern überdies atraumatisch wirkt.

Bei einer weiteren bevorzugten Ausführungsform der Erfindung sind die Endmaschen durch Schlaufen gebildet, die entlang der Abschlusskante versetzt angeordnet sind und sich überlappen. Die Schlaufen sind zur Bildung der, insbesondere miteinander verbundenen, äußeren Drahtelemente an der Abschlusskante an verschiedenen Stellen zusammengeführt, die entlang der Abschlusskante einander nachgeordnet sind. Damit wird eine Konfiguration des Geflechtendes erreicht, bei der mehrere Drahtschlaufen ineinander verschachtelt sind und zwar so, dass die Schlaufen an verschiedenen Stellen entlang der Abschlusskante ineinander übergehen. Damit wird eine gestaffelte Schlaufenanordnung erreicht, wobei sich die Staffelung entlang der Abschlusskante erstreckt.

Die Ausbildung der Endmaschen durch die vorstehend beschriebenen Schlaufen bietet eine einfache Realisierungsmöglichkeit des Geflechtendes, die zu einem umlaufenden glatten Rand führt. Außerdem bietet die Schlaufenkonfiguration eine große Variationsbreite, da die Staffelung der Schlaufen auf verschiedene Weise verändert werden kann.

Bei einer weiteren bevorzugten Ausführungsform sind die inneren Drahtelemente der Abschlusskante zugeführt. Alternativ sind die inneren Drahtelemente von der Abschlusskante abgezweigt. Damit sind zwei Möglichkeiten zur Verfügung gestellt, eine Verstärkung der Abschlusskante bei Bildung eines umlaufenden einziehbaren Randes zu erreichen. Bei der ersten Möglichkeit wird ausgehend von einem dünnen Abschnitt der Abschlusskante, beispielsweise mit nur einem einzigen äußeren Drahtelement, durch die Zufuhr der inneren Drahtelemente zur Abschlusskante diese sukzessive verstärkt, so dass die Abschlusskante mit jeder folgenden Endmasche mehr äußere Drahtelemente aufweist und somit dicker wird. Bei der zweiten Möglichkeit wird ausgehend von einer verstärkten Abschlusskante bzw. einem verstärkten Abschnitt der Abschlusskante diese in ihrer Stärke verringert indem von der Abschlusskante Drahtelemente abgezweigt werden, die als sogenannte innere Drahtelemente im Geflecht weiter geführt sind. Bei beiden Alternativen gehen die äußeren Drahtelemente der Abschlusskante in die abgezweigten, bzw. zugeführten inneren Drahtelemente über.

Die ineinander übergehenden äußeren und inneren Drahtelemente ein und derselben Endmasche sind Abschnitte eines durchgehenden Drahtes und können allgemein auch als innere oder äußere Drahtelementabschnitte bezeichnet werden bzw. als Drahtabschnitte.

Beim Übergang der inneren Drahtelemente in die äußeren Drahtelemente bzw. umgekehrt beim Übergang der äußeren Drahtelemente in die inneren Drahtelemente ist die Windungsrichtung und/oder die Richtung der Axialkomponente bezogen auf die Längsachse des Hohlkörpers geändert. Die Richtungsänderung der Drahtelemente vereinfacht flechttechnisch die Zusammenführung der einzelnen Drähte in einer gemeinsamen Abschlusskante.

Die inneren Drahtelemente und die äußeren Drahtelemente können denselben Flechtwinkel aufweisen, wodurch erreicht wird, dass die Abschlusskante und die im Geflecht befindlichen Drähte nicht kollidieren. Außerdem wird durch den gleichen Flechtwinkel vermieden, dass sich die Struktur beim Einziehen in das Zufuhrsystem verzerrt. Dabei ist nicht ausgeschlossen, dass sich der Flechtwinkel im Geflecht ändert. Es genügt, wenn die sich an die Abschlusskante anschließenden inneren Drahtelemente, bzw. die kantennahen inneren Drahtelemente und die äußeren Drahtelemente denselben Flechtwinkel aufweisen.

Die Wiedereinziehbarkeit in das Zufuhrsystem wird insbesondere dann verbessert bzw. erleichtert, wenn alle Drähte bzw. Drahtelemente, die sich auf derselben axialen Höhe befinden, den gleichen Flechtwinkel aufweisen. Das gilt für die inneren Drahtelemente und für die äußere Drahtelemente. Der Flechtwinkel ist der Winkel zwischen dem Drahtelement und einer Projektion der Längsmittelachse der Vorrichtung. Der Winkel kann zwischen 0°, insbesondere mehr als 0°, und 90° betragen. In Abhängigkeit von der Spiralrichtung verschiedener Drahtelemente oder Drahtabschnitte kann der Flechtwinkel bei demselben Absolutwert unterschiedlich orientiert sein, wie bspw. im Bereich der Umlenkung eines Drahtelements.

Vorzugsweise haben alle inneren Drähten und äußeren Drähten im gesamten Geflechtbereich der Abschlusskante den gleichen Winkel. Der Geflechtbereich der Abschlusskante ist als der Bereich der medizinischen Vorrichtung, der sich zwischen zwei Ebenen erstreckt, die einerseits senkrecht zur Achse des Geflechtes und andererseits jeweils durch die Spitze und durch den Scheitel der Abschlusskante verlaufen. Dies ist also der gesamte, in Längsrichtung beschränkte Bereich, zu dem die Abschlusskante gehört. Bevorzugt ist, wenn alle Drähte im Geflechtbereich der Abschlusskante, oder auf einer gleichen Höhe im Geflechtbereich der Abschlusskante, einen ähnlichen Winkel haben (absoluter Wert). Bevorzugt ist der Unterschied zwischen dem Flechtwinkel von zwei Drähten maximal 20° 15° 10° 8° 6° 5° 4° 3° 2° 1°.

An den Umlenkungsstellen, bei denen die innere Drahtelemente in die Abschlusskante übergehen, haben die Drähte einen sich ändernde Flechtwinkel. Die Umlenkungsstelle kann eine Knickstelle aufweisen. Bevorzugt weist die Umlenkungsstelle einen Radius auf, der kleiner als 1 mm, 0,8 mm, 0,6 mm, 0,4 mm, 0,3 mm, 0,2 mm 0,1 mm ist. Dadurch kann der Draht auf einem kurzen Weg die Spiralrichtung ändern. Bevorzugt haben die Drähte, bevorzugt alle Drähte, die umgelenkt werden, vor und nach der Umlenkungsstelle, also vor und nach dem Radius oder der Knickstelle, einen Flechtwinkelunterschied (absoluter Wert) von höchstens 20° 15° 10° 8° 6° 5° 4° 2° 1°.

Die miteinander verbundenen äußeren Drahtelemente der Endmaschen können formschlüssig, insbesondere durch Verdrillen und/oder Verflechten, und/oder stoffschlüssig, insbesondere durch Verkleben, Verschweißen oder Verlöten, und/oder durch mechanische Verbindungsmittel, insbesondere durch Coils und/oder Hülsen und/oder Drähte verbunden sein. Bei der Verbindungstechnik sind Hülsen, bevorzugt röntgensichtbare, bevorzugt Platin-Iridium Hülsen von Vorteil. In einer bevorzugten Variante werden die Hülsen mechanisch gecrimpt. Die Hülsen können aber auch geschweißt werden. Das Anbringen von röntgensichtbarem Material auf der Abschlusskante, evtl. auf der gesamten Kante, hat den Vorteil, dass so das offene Geflechtende und dessen Position gut sichtbar sind.

Bei einer bevorzugten Ausführungsform der Erfindung nimmt die Anzahl der äußeren Drahtelemente des ersten Abschnitts und die Anzahl der äußeren Drahtelemente des zweiten Abschnitts jeweils zu einem in Längsrichtung des Hohlkörpers vorderen Bereich der Abschlusskante zu. Die Anzahl der äußeren Drahtelemente des ersten Abschnitts und die Anzahl der äußeren Drahtelemente des zweiten Abschnitts sind jeweils im vorderen Bereich maximal. Der vordere Bereich der Abschlusskante ist beispielsweise bei einer bezogen auf die Längsrichtung des Hohlkörpers schräg angeordneten Abschlusskante der Bereich der vorderen Spitze bzw. der proximale Bereich der Abschlusskante. Die Anzahl der äußeren Drahtelemente nimmt mit jeder zusätzlichen Endmasche ausgehend von einer vom vorderen Bereich entfernt angeordneten Endmasche zu und zwar in Richtung des vorderen Bereichs. Dort ist die Anzahl der äußeren Drahtelemente maximal. Dies gilt für jeden der beiden Abschnitte. Durch diese Ausführungsform wird eine im vorderen Bereich der Abschlusskante stabile Struktur geschaffen, die die Führungsfunktion der Abschlusskante beim Einziehen in das Zufuhrsystem verbessert.

Die äußeren Drahtelemente der Endmaschen können in einer vorderen Endmasche zusammengeführt sein. Auch dadurch wird die Stabilität der Abschlusskante im vorderen Bereich erhöht. Die äußeren Drahtelemente können eine einzige Gruppe bilden, die im Bereich der vorderen Endmasche umgelenkt ist. Dies bedeutet, dass die freien Drahtenden der jeweiligen Drähte, die die äußeren Drahtelemente bilden, im Bereich des anderen Geflechtendes angeordnet sind. Dort können die freien Drahtenden auf herkömmliche Weise fixiert sein. Es ist auch möglich, die freien Drahtenden wie in der auf die Anmelderin zurückgehenden DE 10 2009 006 180.0 anzuordnen. Der Inhalt dieser Anmeldung wird durch Verweis vollumfänglich aufgenommen.

Alternativ können die äußeren Drahtelemente wenigstens zwei Gruppen bilden, die im Bereich der vorderen Endmasche verbunden sind. Dies bedeutet, dass die freien Enden der zugehörigen Drähte bzw. derjenigen Drähte, die die jeweiligen äußeren Drahtelemente bilden, im Bereich der vorderen Endmasche zusammengeführt und dort verbunden sind. Die Verbindung der freien Drahtenden kann auf herkömmliche Weise erfolgen.

Vorzugsweise nimmt die Anzahl der äußeren Drahtelemente des ersten Abschnitts und des zweiten Abschnitts jeweils zu einem in Längsrichtung des Hohlkörpers vorderen Bereich der Abschlusskante hin ab und ist im vorderen Bereich minimal. Im Hinblick auf die Anordnung des vorderen Bereichs wird auf die vorstehenden Ausführungen verwiesen. Im Unterschied zu der vorgenannten Ausführungsform nimmt die Anzahl der Drahtelemente in proximaler Richtung der Abschlusskante ab. Mit anderen Worten steigt die Anzahl der äußeren Drahtelemente ausgehend vom vorderen Bereich der Abschlusskante mit zunehmendem Abstand, bis die Anzahl der äußeren Drahtelemente maximal ist. Die äußeren Drahtelemente sind zu einem Drahtbündel zusammengefasst, das zur Verstärkung des Hohlkörpers im Geflecht angeordnet ist und einen Teil des Geflechts bildet. Dies bedeutet, dass das die Abschlusskante bildende Drahtbündel in das Geflecht hineingeführt ist und einen Teil des Geflechtes bildet. Durch die damit erreichte Verstärkung des Geflechtes wird der Hohlkörper stabilisiert.

Bei einer weiteren bevorzugten Ausführungsform der Erfindung ist zwischen dem vorderen Bereich und einem hinteren Bereich der Abschlusskante ein Zwischenbereich angeordnet. Die Anzahl der äußeren Drahtelemente nimmt, ausgehend vom Zwischenbereich, jeweils zum vorderen Bereich und zum hinteren Bereich der Abschlusskante hin ab. Die Anzahl der äußeren Drahtelemente ist im Zwischenbereich maximal. Der hintere Bereich der Abschlusskante ist derjenige Bereich, in dem die Abschlusskante in die geschlossene Wandung des Geflechts übergeht. Bei einer schräg angeordneten Abschlusskante ist der hintere Bereich der Abschlusskante gegenüber dem vorderen Bereich angeordnet und von diesem beabstandet, wobei der vordere Bereich eine Geflechtspitze bildet. Dies gilt für alle Ausführungsbeispiele, bei denen die Abschlusskante schräg angeordnet ist. Bei der vorstehend genannten Ausführungsform ist der Zwischenbereich zwischen dem vorderen und dem hinteren Bereich der Abschlusskante angeordnet und jeweils von diesen beabstandet. Die damit verbundene seitlich Verstärkung der Abschlusskante eröffnet weitere Einsatzmöglichkeiten der Vorrichtung.

Bei einer anderen Ausführungsform der Erfindung bilden die inneren Drahtelemente innere Maschengrenzen der Endmaschen des ersten Abschnitts und/oder des zweiten Abschnitts, die sich ausgehend von einer Abschlusskante ins Geflechtinnere erstrecken, wobei die Endmaschen zumindest teilweise an den inneren Maschengrenzen miteinander verbunden sind. Die Verbindung der Endmaschen untereinander erfolgt bei dieser Ausführungsform hauptsächlich durch die inneren Drahtelemente und weniger durch die äußeren Drahtelemente. Es hat sich gezeigt, dass auch bei einer solchen Ausgestaltung der Erfindung ein ausreichend stabile Abschlusskante erreicht wird, die ein Wiedereinziehen des Geflechtendes in ein Zufuhrsystem ermöglicht, ohne dass die einzelnen Endmaschen mit der Eintrittöffnung des Zufuhrsystems verkanten.

Dabei können die äußeren Drahtelemente der Endmaschen so angeordnet sein, dass diese nicht überlappen. Bei dieser Ausführungsform können zwischen den einzelnen Endmaschen Kanten entstehen, die aber für das Wiedereinziehen des Hohlkörpers in das Zufuhrsystem unschädlich sind, da sich diese in der Einzugsrichtung erstrecken bzw. nicht entgegen der Einzugsrichtung vorstehen.

Es wird darauf hingewiesen, dass sich im Allgemeinen entlang der Abschlusskante kleine Vorsprünge bilden können. Diese können beispielsweise durch die Verdrillung der Drähte bedingt sein. Im Rahmen der Erfindung wird die Abschlusskante als glatt bezeichnet, wenn sich ein im Wesentlichen kontinuierlicher Verlauf der Drahtelemente einstellt. Das bedeutet, dass keine scharfen Kanten entstehen, die das Wiedereinziehen des Gittergeflechts in ein Zufuhrsystem behindern. Im Bereich der Abschlusskante können also einzelne Drahtelemente vorstehen, wobei diese Drahtelemente eine Krümmung beschreiben, die einen derart großen Radius beschreibt, dass die Abschlusskante insgesamt glatt bzw. kontinuierlich glatt ausgebildet ist. Vorteilhafterweise steht das vorstehende Drahtelement höchstens um einen Betrag vor, der maximal dem Durchmesser des Drahtelements entspricht. In besonderen Fällen ist es auch möglich, dass das Drahtelement über benachbarte Drahtelemente vorsteht, wobei der Vorstand höchstens dem dreifachen Drahtdurchmesser entspricht. Wesentlich ist, dass die Abschlusskante derart kontinuierlich glatt geformt ist, dass das Wiedereinziehen in ein Zufuhrsystem ermöglicht ist.

Bei einer weiteren Ausführungsform können die Endmaschen des ersten Abschnitts und/oder des zweiten Abschnitts jeweils wenigstens zwei Schlaufen aufweisen, die entlang der Abschlusskante nebeneinander angeordnet sind, wobei wenigstens eine dritte Schlaufe die erste und die zweite Schlaufe überlappt. Diese Ausführungsform hat den Vorteil, dass sich durch die verschieden kombinierbaren Schlaufen ein Geflecht herstellen lässt, das im Bereich der Abschlusskante unterschiedlich ausgelegt wird derart, dass die mechanischen Eigenschaften, wie die Radialkraft, die Flexibilität und die Feinmaschigkeit sehr genau eingestellt werden können. Dies gilt nicht nur für den Bereich nahe der Außenkante, sondern auch für das gesamte Geflecht.

Bei einer besonders bevorzugten Ausführungsform der Erfindung bildet wenigstens ein äußeres Drahtelement und/oder ein zusätzliches Drahtelement eine Verlängerung, die sich über die Kontur der Abschlusskante hinaus erstreckt. Die Verlängerung kann beispielsweise dazu genutzt werden, einen Führungsdraht mit dem Hohlkörper zu verbinden. Die Verlängerung kann beispielsweise in der Form einer Schlaufe ausgebildet sind, in die ein entsprechendes Gegenstück des Führungsdrahtes einhakt.

Die vorstehend genannten Merkmale der verschiedenen Ausführungsformen sind konstruktive Merkmale, die an der Vorrichtung nachvollzogen werden können und diese kennzeichnen. Die vorstehend genannten Merkmale der verschiedenen Ausführungsformen werden auch, soweit sinnvoll, im Zusammenhang mit dem Herstellungsverfahren als Verfahrensmerkmale offenbart.

Die Erfindung wird nachfolgend anhand von Ausführungsbeispielen unter Bezugnahme auf die beigefügten schematischen Zeichnungen mit weiteren Einzelheiten näher erläutert. In diesen zeigen:
- Fig. 1: eine perspektivische Seitenansicht einer medizinischen Vorrichtung nach einem erfindungsgemäßen Ausführungsbeispiel, insbesondere einen Korb;
- Fig. 2: eine Abwicklung der Vorrichtung gemäß Fig. 1;
- Fig. 3: einen vergrößerten Ausschnitt der Abschlusskante der Vorrichtung gemäß Fig. 2;
- Fig. 3a: eine schematische Ansicht zur Darstellung der Änderung der Wicklungsrichtung der Drahtelemente der Vorrichtung gemäß Fig. 1;
- Fig. 3b: eine schematische Ansicht zur Darstellung der Änderung der Richtung der Axialkomponente der Drahtelemente bei der Vorrichtung gemäß Fig. 4;
- Fig. 4: die Abwicklung einer medizinischen Vorrichtung nach einem weiteren erfindungsgemäßen Ausführungsbeispiel;
- Fig. 5: die Vorrichtung gemäß Fig. 1 mit weiteren Einzelheiten im Bereich der Abschlusskante;
- Fig. 6a: eine perspektivische Seitenansicht einer Vorrichtung nach einem weiteren erfindungsgemäßen Ausführungsbeispiel;
- Fig. 6b: eine perspektivische Seitenansicht einer Vorrichtung nach einem weiteren erfindungsgemäßen Ausführungsbeispiel;
- Fig. 7: eine schematische Darstellung einer Anordnung umfassend die Vorrichtung gemäß Fig. 6a oder Fig. 6b und ein zugehöriges Zufuhrsystem;
- Fig. 8: eine Vergrößerung des Verbindungsbereichs zwischen dem Zufuhrsystem und der Vorrichtung gemäß Fig. 7;
- Fig. 9: eine schematische Darstellung einer medizinischen Vorrichtung nach einem weiteren erfindungsgemäßen Ausführungsbeispiel im Gebrauch;
- Fig. 10: die Abwicklung einer medizinischen Vorrichtung nach einem weiteren erfindungsgemäßen Ausführungsbeispiel;
- Fig. 11: die Abwicklung einer medizinischen Vorrichtung nach einem weiteren erfindungsgemäßen Ausführungsbeispiel mit einer alternativen Schlaufenkonfiguration;
- Fig. 12: die Abwicklung einer medizinischen Vorrichtung nach einem weiteren erfindungsgemäßen Ausführungsbeispiel, bei dem die inneren Maschengrenzen verbunden sind;
- Fig. 13: die Abwicklung einer medizinischen Vorrichtung nach einem weiteren erfindungsgemäßen Ausführungsbeispiel, bei dem die Schlaufen teilweise nebeneinander und teilweise überlappend angeordnet sind;
- Fig. 14a: die Abwicklung einer medizinischen Vorrichtung nach einem weiteren erfindungsgemäßen Ausführungsbeispiel mit umgelenkten ersten Drahtelementen;
- Fig. 14b: die Abwicklung einer medizinischen Vorrichtung nach einem weiteren erfindungsgemäßen Ausführungsbeispiel mit nicht-umgelenkten ersten Drahtelementen;
- Fig. 15a: die Abwicklung einer medizinischen Vorrichtung nach einem weiteren erfindungsgemäßen Ausführungsbeispiel mit gefachten Einzeldrähten und umgelenkten ersten Drahtelementen;
- Fig. 15b: die Abwicklung einer medizinischen Vorrichtung nach einem weiteren erfindungsgemäßen Ausführungsbeispiel mit gefachten Einzeldrähten und nicht-umgelenkten ersten Drahtelementen; und
- Fig. 16: die Abwicklung einer medizinischen Vorrichtung nach einem weiteren erfindungsgemäßen Ausführungsbeispiel mit einer Mehrfachdrahtführung;

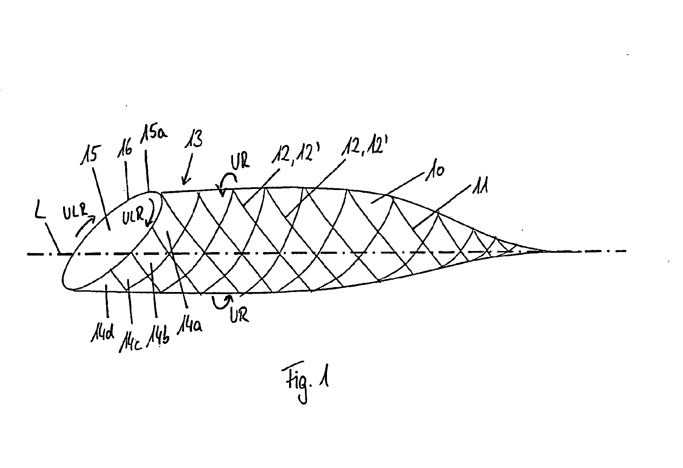

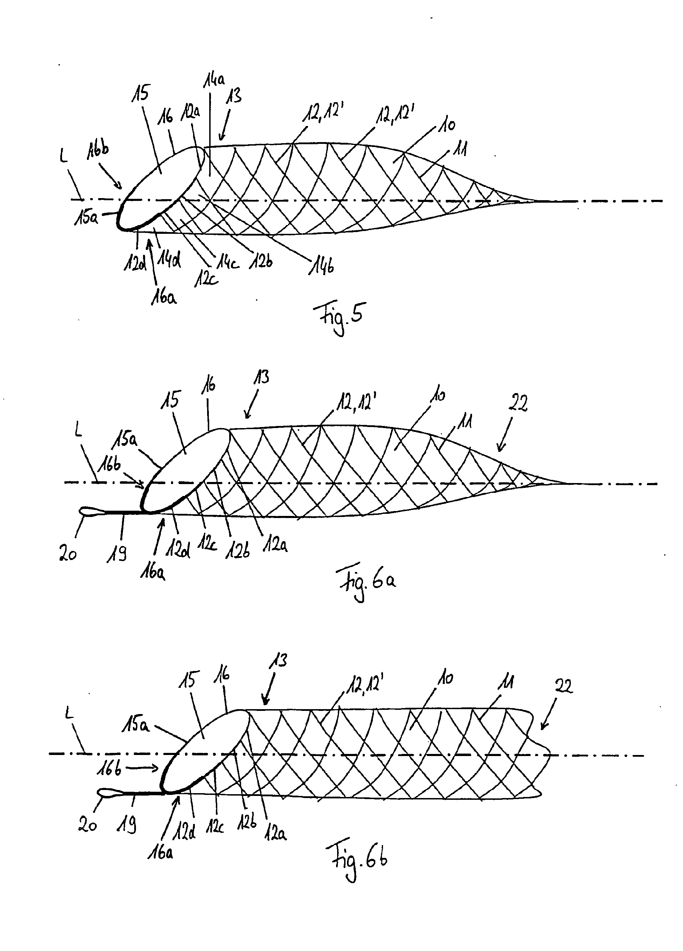

In den Fig. 1 bis 3 sowie in Fig. 5 ist eine medizinische Vorrichtung zur Einfuhr in ein Hohlorgan, insbesondere in ein menschliches Hohlorgan, nach einem erfindungsgemäßen Ausführungsbeispiel dargestellt. Bei der Vorrichtung handelt es sich um einen Korb, der beispielsweise zur Thrombenentfernung eingesetzt werden kann. Die Erfindung ist auch auf andere medizinische Vorrichtungen anwendbar. Beispielsweise genannt seien Stents, Strömungsteiler, Filter und dergleichen. Insbesondere bei stentartigen Systemen, die zu einer Beeinflussung der Strömung eingesetzt werden, ist die Vorrichtung geeignet. Systeme zur Beeinflussung der Strömung sind besondere feinmaschig und weisen kleine Zellen auf. Zum Beispiel weist eine feinmaschige Vorrichtung über 16, 24 32, 36, 40, 44, 48, 64, 72, 80, 96 Drahtelemente auf. Bei solchen Vorrichtungen ist die genaue Positionierung wichtig. Zum Beispiel soll die Vorrichtung die Strömung in einem Aneurysma oder an einer artero-venöse Malformation beeinflussen, bzw. verringern, während Seitenäste offen bleiben sollen. Es ist wichtig, dass die Vorrichtung wieder in den Katheter zurückgezogen wird, wenn die Positionierung nicht optimal ist, zum Beispiel die Strömungsbeeinflussung unzureichend ist oder Seitenäste teilweise verschlossen sind. Die Strömungsbeeinflussung und der Verschluss von Seitenästen kann durch eine angiografische Kontrolle evaluiert werden. Die Vorrichtung kann auch ein teilweise bespanntes oder vollständig bespanntes Implantat sein. Eine Membran, bevorzugt eine Kunststoffmembran, kann mindestens einen Bereich der Vorrichtung teilweise oder vollständig abdecken. Bei vollständiger Abdeckung spricht man von einem Stent-Graft. Die vollständige Abdeckung kann zum Beispiel durch eine dünne Polyuretahnfolie erfolgen. Zu diesem Zweck kann das Geflecht zum Beispiel in einem Polyurethanmischung getaucht werden. Das bespannte Implantat dient dazu, die Blutströmung teilweise oder vollständig zu unterbinden, zum Beispiel bei Aneurysmen oder arterovenösen Malformationen.

Die Erfindung ist generell anwendbar auf Implantate oder auf medizinische Geräte, die temporär im Körper freigesetzt werden, und bei denen es auf eine Wiedereinziehbarkeit in das entsprechende Zufuhrsystem ankommt. Die Wiedereinziehbarkeit kann beispielsweise zur Repositionierung, insbesondere bei Implantaten, wie Stents, oder generell beim Zurückholen temporär freigesetzter medizinischer Geräte eine Rolle spielen.

Die Vorrichtung gemäß Fig. 1 umfasst einen Hohlkörper 10 mit einer Längsachse L. Der Hohlkörper 10 ist bei dem Beispiel gemäß Fig. 1 rotationssymmetrisch, insbesondere zylindrisch ausgebildet. Andere geometrische Formen des Hohlkörpers, auch nicht rotationssymmetrische Formen, sind möglich. Der Hohlkörper weist eine Wandung auf, die in der Form eines Geflechts 11 aus Drahtelementen 12 gebildet ist. Die Wandung ist geschlossen insofern als diese vollumfänglich das Lumen des Hohlkörpers umgibt und begrenzt. Die Drahtelemente 12 können Metalldrähte oder Kunststoffdrähte sein. Generell handelt es sich bei den Drahtelementen 12 um Filamente oder fadenförmige Elemente, die zum Flechten geeignet sind. Als Material für die Drahtelemente kommen alle gängigen Materialien in Frage, die üblicherweise für Implantate oder andere in ein menschliches Hohlorgan einzuführende medizinische Geräte geeignet sind.

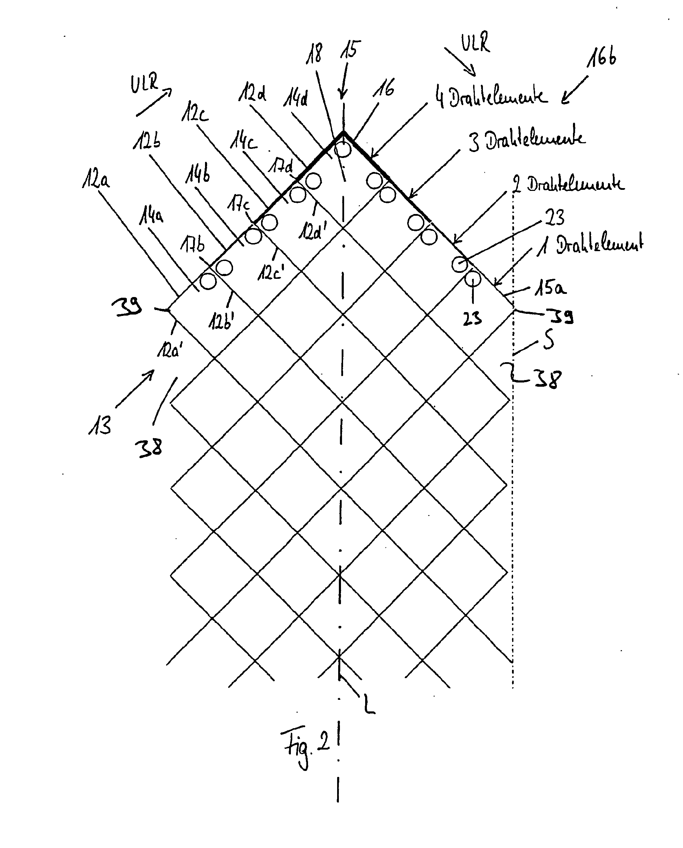

Das Geflecht weist, wie in Fig. 1 zu erkennen, eine erste Reihe 13 von nebeneinander angeordneten Endmaschen 14a, 14b, 14c, 14d auf. Die erste Reihe 13 der Endmaschen 14a, 14b, 14c, 14d ist auch in Fig. 2 gut zu erkennen. Die Endmaschen 14a, 14b, 14c, 14d begrenzen ein axiales Geflechtende 15. Bei dem Korb gemäß Fig. 1 ist das durch die Endmaschen 14a, 14b, 14c, 14d begrenzte axiale Geflechtende 14 offen. Das andere Geflechtende ist bei dem Korb geschlossen. Es ist auch möglich, insbesondere bei Implantaten wie Stents, die beiden axialen Geflechtenden erfindungsgemäß auszubilden. Generell soll zumindest das wiedereinziehbare oder proximale Geflechtende erfindungsgemäß ausgebildet sein.

Die erste Reihe 13 mit den nebeneinander angeordneten Endmaschen 14a, 14b, 14c, 14d setzt sich in Richtung der Längsachse in der Form weiterer Maschenreihen fort, die zusammen das Geflecht 11 bilden. Die weiteren Maschenreihen werden in an sich bekannter Weise hergestellt bzw. geflochten. Die Endmaschen 14a, 14b, 14c, 14d der ersten Reihe 13 weisen äußere Drahtelemente 12a bis 12d auf, die zusammen die Abschlusskante 16 des Geflechtendes 15 bilden. Die äußeren Drahtelemente 12a, 12b, 12c, 12d sind also alle in Längsrichtung L außen an der Geflechtkante angeordnet.

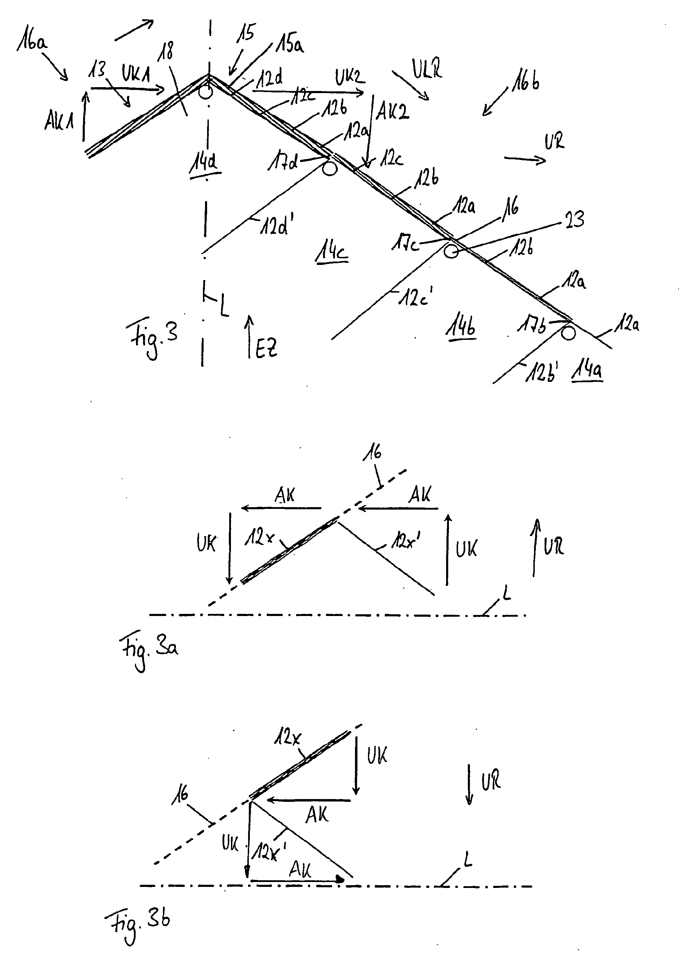

Der Verlauf bzw. die Anordnung der ersten und zweiten Axialkomponente AK1, AK2 ist in Fig. 3 dargestellt, ebenso wie der Verlauf der beiden Umfangskomponenten UK1, UK2. In Fig. 3 sind der erste und zweite Abschnitt 16a der Abschlusskante 16 dargestellt, wobei nur der rechte Abschnitt 16b der Abschlusskante 16 vollständig dargestellt ist. Vom linken Abschnitt 16a der Abschlusskante 16 sind die in der vorderen Endmasche 18 zusammengeführten äußeren Drahtelemente 12a, 12b, 12c, 12d dargestellt. Die übrigen Endmaschen 14a, 14b, 14c entsprechen den im Zusammenhang mit dem zweiten (in Fig. 3b rechten) Abschnitt 16b dargestellten Endmaschen 14a, 14b, 14c. Wie in Fig. 3 zu erkennen, weist jede der beiden Abschlusskanten 16a, 16b jeweils mehrere äußere Drahtelemente 12a, 12b, 12c, 12d auf, die zusammen einen umlaufenden Rand 15a der Abschlusskante 16 bilden. Der umlaufende Rand ist bei dem Ausführungsbeispiel gemäß Fig. 3 kantenlos ausgebildet und erleichtert somit das Einziehen des Geflechtendes 15 in ein Zufuhrsystem. Es ist nicht ausgeschlossen, dass der Rand 15a Kanten aufweist, die sich in distaler Richtung erstrecken, d.h. vom Anwender weg erstrecken und somit nicht entgegen der Einzugsrichtung vorstehen. Die Einzugsrichtung ist mit dem Pfeil EZ in Fig. 3 dargestellt. Dort ist weiter zu erkennen, dass die Einzugsrichtung EZ in Längsrichtung L des Hohlkörpers verläuft.

Sowohl der erste Abschnitt 16a als auch der (komplett dargestellte) zweite Abschnitt 16b der Abschlusskante 16 ist jeweils aus mehreren äußeren Drahtelementen 12a, 12b, 12c, 12d gebildet, die entlang der Abschlusskante 16 einander unmittelbar nachgeordnet sind. Im vorliegenden Ausführungsbeispiel sind die äußeren Drahtelemente 12a, 12b, 12c, 12d fluchtend in einer Linie angeordnet. Es ist auch möglich, dass die äußeren Drahtelemente 12a, 12b, 12c, 12d auf der Abschlusskante 16 nach außen bzw. nach innen leicht versetzt zueinander angeordnet sind, wobei die dabei bildenden Kanten nicht entgegen der Einzugsrichtung EZ vorstehen.

Die Drahtelemente 12a, 12b, 12c, 12d sind einander unmittelbar nachgeordnet dadurch, dass pro Endmasche 14a, 14b, 14c, 14d jeweils ein inneres Drahtelement 12a', 12b', 12c', 12d' der Abschlusskante zugeführt und in diese integriert ist. Dadurch ergibt sich die sukzessive Anordnung zusätzlicher äußerer Drahtelemente 12a, 12b, 12c, 12d in Richtung der vorderen Endmasche 18. Dieses Prinzip gilt für alle Ausführungsbeispiele dieser Anmeldung, wobei die äußeren Drahtelemente 12a, 12b, 12c, 12d unabhängig davon, ob diese im Verlauf der Abschlusskante 16 sich überlappen oder nicht, im Wesentlichen in einer Reihe entlang der Abschlusskante 16 angeordnet sind.

In Fig. 3 ist ferner die erste Axialkomponente AK1 des ersten (in Fig. 3 linken) Abschnitts 16a dargestellt. In Umlaufrichtung ULR gesehen, erstreckt sich die erste Axialkomponente AK1 des ersten Abschnitts 16a in proximaler Richtung bzw. in Einzugrichtung EZ. In derselben Umlaufrichtung ULR gesehen, erstreckt sich die zweite Axialkomponente AK2 des zweiten Abschnitts 16b entgegen der Einzugrichtung. Das bedeutet, dass beide Axialkomponenten AK1, AK2 in Längsrichtung L des Hohlkörpers verlaufen, wobei die, in derselben Umlaufrichtung ULR gesehene Richtung der Axialkomponenten AK1, AK2 entgegengesetzt ist.

Durch die einheitliche Orientierung der äußeren Drahtelemente 12a, 12b, 12c, 12d des ersten Abschnitts 16a bzw. des zweiten Abschnitts 16b mit entgegengesetzten Axialkomponenten AK1, AK2 wird eine Form der Abschlusskante 16 erreicht, die ein Wiedereinziehen des Geflechtendes in das Zufuhrsystem ermöglicht. Auf diese Weise ist es möglich, einen glatten Rand 15a der Abschlusskante 16 zu bilden, der widerstandsfrei in das Zufuhrsystem hineingleitet. Die Orientierung der äußeren Drahtelemente 12a, 12b, 12c, 12d mit entgegengesetzten Axialkomponenten AK1, AK2 lässt auch die Bildung von Kanten zu, die sich in distaler Richtung erstrecken und somit ein Einziehen in das Zufuhrsystem ebenfalls nicht behindern.

Die einheitliche Orientierung der äußeren Drahtelemente 12a, 12b, 12c, 12d des jeweiligen Abschnitts 16a, 16b schließt ebenfalls nicht aus, dass die Axialkomponenten der einzelnen äußeren Drahtelemente 12a, 12b, 12c, 12d unterschiedlich groß sind, so dass eine gekrümmte Ebene, in der die Abschlusskante bzw. die Öffnung des axialen Geflechtendes liegt, erreicht wird. Im Ausführungsbeispiel gemäß Fig. 3 sind die Axialkomponenten AK1 bzw. AK2 jeweils gleich groß. Dadurch ergibt sich eine gerade Ebene, in der die Abschlusskante 16 liegt, wie in Fig. 1 dargestellt.

Ferner ist in Fig. 3 zu erkennen, dass die Umfangskomponenten UK1, UK2 der äußeren Drahtelemente 12a, 12b, 12c, 12d der beiden Abschnitte 16a, 16b in derselben Richtung verlaufen und zwar in Umfangsrichtung UR des Hohlkörpers gesehen. Zum Verlauf der Umfangsrichtung UR wird auf die Darstellung gemäß Fig. 1 verwiesen, in der die Umfangsrichtung ebenfalls eingezeichnet ist. Die Unterschiede zwischen der Umfangsrichtung und der Umlaufrichtung einmal bezogen auf die Wandung und bezogen auf den Rand 15a werden ebenfalls aus Fig. 1 deutlich.

Der Verlauf der beiden Axialkomponenten AK1, AK2 bzw. der beiden Umfangskomponenten UK1, UK2, wie anhand Fig. 3 beschrieben, gilt für alle Ausführungsbeispiele dieser Anmeldung.

Die Abschlusskante 16 kann, wie in Fig. 1 dargestellt, schräg bezogen auf die Längsachse L angeordnet sein. Der Neigungswinkel ist dabei beliebig einstellbar und zwar über eine Variation des Flechtwinkels. Die Abschlusskante 16 begrenzt eine ebene Öffnung des Geflechtendes 15. Es ist auch möglich, dass die Abschlusskante 16 eine gekrümmte Kontur aufweist, insbesondere eine konkav oder konvex gekrümmte Kontur. Dies gilt für alle in der Anmeldung beschriebenen Ausführungsbeispiele.

Der Aufbau der Abschlusskante 16 ist besonders gut in den Fig. 2 und 3 zu erkennen. Das Konfigurationsprinzip der Abschlusskante 16 beruht darauf, dass sich wenigstens ein erstes äußeres Drahtelement 12 a einer Endmasche 14a in dem Bereich einer anderen Endmasche 14b, 14c, 14d erstreckt. Im Bereich der anderen Endmasche 14b, 14c, 14d ist wenigstens ein zweites äußeres Drahtelement 12b, 12c, 12d vorgesehen, also ein zusätzliches Drahtelement, das sich zusammen mit dem ersten äußeren Drahtelemente 12a entlang der Abschlusskante 16 erstreckt und mit dem ersten äußeren Drahtelement 12a verbunden ist. Dadurch wird erreicht, dass die Endmaschen untereinander verbunden sind und die Abschlusskante einen glatten und festen Rand aufweist. Es ist auch möglich, die Drahtelemente 12a, 12b, 12c, 12d lose, d.h. unverbunden entlang der Abschlusskante zu führen.

Die Drähte 12a, 12b, 12c, 12d müssen nicht auf der gesamten Länge miteinander verbunden sein. Eine leichte Verdrillung, die es ermöglicht, dass die Drähte 12a, 12b, 12c, 12d relativ zueinander beweglich sind, ist auch möglich. Zum Beispiel wickelt sich der Draht 12a nur einmal um den Draht 12b auf Höhe der Masche 12b. Das bewirkt, dass die Bewegung, insbesondere die Komprimierung beider Endschlaufen 14a und 14b nicht unabhängig voneinander stattfinden kann. Beide Schlaufen greifen ineinander ein.

Wie in Fig. 2 dargestellt, weist die äußere (linke) erste Endmasche 14a, die der Schnittlinie S der Abwicklung gegenüberliegt, ein einziges äußeres Drahtelement auf, das einen ersten Abschnitt der Abschlusskante 16 bildet. Es ist auch möglich, dass die erste Endmasche 14a mehr als ein äußeres Drahtelement 12a, beispielsweise 2, 3 oder mehr äußere Drahtelemente 12a aufweist. Ferner kann im Bereich jeder neuen Endmasche 14a, 14b, 14c, 14d mehr als ein einziges Drahtelement, z.B. 2 oder 4 Elemente, hinzukommen.

Das äußere Drahtelemente 12a der ersten Endmasche 14a bzw. der Gruppe von Drahtelementen 12a der ersten Endmasche 14a erstreckt sich in den Bereich der angrenzenden zweiten Endmasche 14b. Die zweite Endmasche 14b weist ein zusätzliches zweites äußeres Drahtelement 12b auf, das mit dem ersten äußeren Drahtelement 12a der ersten Endmasche verbunden ist. Dadurch weist die Abschlusskante 16 im Bereich der zweiten Endmasche 14a eine erhöhte Dicke auf, wie durch den stärkeren Strich in Fig. 2 dargestellt. Die beiden ersten und zweiten äußeren Drahtelemente 12a, 12b erstrecken sich in den Bereich der nachgeordneten dritten Endmasche 14c und sind dort mit einem dritten äußeren Drahtelement 12c verbunden. Die drei äußeren Drahtelemente 12a, 12b, 12c der dritten Endmasche 14c erstrecken sich in dem Bereich der vierten Endmasche 14d und sind dort mit einem weiteren äußeren Drahtelement 12d verbunden, so dass die vierte Endmasche 14d vier äußere Drahtelemente aufweist. Dieses Prinzip ist auf jede beliebige Anzahl von Endmaschen anwendbar. Dies gilt für alle Ausführungsbeispiele dieser Anmeldung.

Somit nimmt allgemein die Anzahl der äußeren Drahtelemente in Umfangsrichtung und, bei schräg angeordneter Abschlusskante 16, zur Spitze hin zu. Bei dem Ausführungsbeispiel gemäß Fig. 2 weist die erste Endmasche ein Drahtelement und die in Umfangsrichtung nachgeordneten Endmaschen jeweils ein zusätzliches Drahtelement auf, so dass die vierte Endmasche 14d vier Drahtelemente 12a, 12b, 12c, 12d umfasst, wie in Fig. 2 dargestellt. Aus der Erhöhung der Anzahl der äußeren Drahtelemente 12a, 12b, 12c, 12d pro Endmasche ergibt sich, dass die einzelnen Endmaschen 14a, 14b, 14c, 14d jeweils eine unterschiedliche Anzahl von äußeren Drahtelementen 12a, 12b, 12c, 12d aufweisen.

Die Drahtelemente der jeweiligen Endmaschen 14a, 14b, 14c, 14d sind miteinander verbunden, beispielsweise verdrillt oder verflochten. Andere Verbindungsmöglichkeiten, insbesondere stoffschlüssige oder durch mechanische Verbindungsmittel sind möglich. Damit wird erreicht, dass die Abschlusskante 16 einen stabilen Rand bildet.

Wie in Fig. 2 dargestellt, ist die Anordnung der äußeren Drahtelemente 12a, 12b, 12c, 12d entlang der Abschlusskante 16 symmetrisch bezogen auf die Längsachse L (siehe Fig. 1).

Die Bildung der Endmaschen kann beispielsweise durch Schlaufen erfolgen, die sich entlang der Abschlusskante 16 überlappen, bzw. die entlang der Abschlusskante 16 versetzt angeordnet sind. Bei dem Ausführungsbeispiel gemäß Fig. 2 ist beispielsweise die von der ersten Endmasche 14a ausgehende Schlaufe die erste Schlaufe, die sich bis zur vorderen Endmasche 18 erstreckt. Die nachgeordnete zweite Schlaufe bildet zusammen mit der ersten Schlaufe die erste, in Fig. 2 linke, Endmasche 14a und. Zur Bildung der ersten Endmasche 14a sind die beiden Schlaufen an der Stelle 17b zusammengeführt und bilden zusammen im Bereich der Abschlusskante 16 die ersten und zweiten äußeren Drahtelemente 12a, 12b. Auf analoge Weise sind die zweiten, dritten und vierten Endmaschen 14b, 14c, 14d und mögliche weitere Endmaschen gebildet. Die vorstehende Flechtkonfiguration ist auch in Fig. 3 in dem dort gezeigten Ausschnitt gut erkennbar. Insbesondere ist gut erkennbar, dass die Anzahl der äußeren Drahtelemente 12a, 12b, 12c, 12d sich entlang der Abschlusskante 16 ändert, insbesondere zur Spitze hin zunimmt.

Das bedeutet, dass die Schlaufen gestaffelt an den in Umfangsrichtung nachgeordneten Stellen 17b, 17c und 17d zusammengeführt sind. Die Schlaufen können symmetrisch bezogen auf die Längsachse L angeordnet sein derart

Andere Schlaufenkonfigurationen sind möglich, die eine entlang des Umfangs der Abschlusskante 16 unterschiedliche Anzahl von äußeren Drahtelementen 12a, 12b, 12c, 12d maschenweise bewirkt.

Wie in den Fig. 1 und 2 weiter zu erkennen, weisen die Endmaschen 14a, 14b, 14c, 14d im Geflecht 11 angeordnete innere Drahtelemente 12a', 12b', 12c' und 12d' auf. Die inneren Drahtelemente 12a', 12b', 12c', 12d' sind in das Geflechtinnere von der Abschlusskante 16 weggeführt und bilden das Geflecht 11. Wie in den Fig. 2 und 3 zu erkennen, sind die inneren Drahtelemente 12a', 12b', 12c' und 12d' umgelenkt und gehen in die äußeren Drahtelemente 12a, 12b, 12c, 12d über. Ein inneres Drahtelement 12a' und das zugehörige äußere Drahtelement 12a gehören also zum selben Draht und bilden unterschiedlich orientierte Abschnitte dieses Drahtes.

Die inneren Drahtelemente 12a', 12b', 12c', 12d' und äußeren Drahtelemente 12a, 12b, 12c, 12d bilden zusammen die vorstehend beschriebenen Schlaufen.

Beim Übergang der inneren Drahtelemente 12a', 12b', 12c' und 12d' in die äußeren Drahtelemente 12a, 12b, 12c, 12d gemäß den Fig. 1 bis 3 ändern die inneren Drahtelemente 12a', 12b', 12c' und 12d' die Windungsrichtung. Dieser Zusammenhang ist in Fig. 3a schematisch verdeutlicht. Dort ist strichpunktiert die Abschlusskante 16 eingezeichnet, sowie ein geflechtbildendes Drahtelement 12x', das durch Umlenkung in ein kantenbildendes Drahtelement 12x übergeht. Die Bezeichnungen "kantenbildendes Drahtelement" und "äußeres Drahtelement" entsprechen sich. Ebenfalls entsprechen sich die Bezeichnungen "inneres Drahtelement" und "geflechtbildendes Drahtelement".

Wie in Fig. 3a ersichtlich, ändert das geflechtbildende Drahtelement 12x' beim Übergang in das kantenbildende Drahtelement 12x die Windungsrichtung und damit seine Umfangsrichtung. Konkret ändert sich die in Umfangsrichtung UR der Wandung verlaufende Umfangskomponente UK des Drahtelements 12x'. Das bedeutet, dass die inneren Drähte 12a', 12b', 12c' und 12d' bzw. die geflechtbildenden Drähte 12x' beispielsweise im Uhrzeigersinn verlaufen und die äußeren Drahtelemente 12a, 12b, 12c, 12d bzw. die kantenbildenden Drahtelemente 12x entgegen dem Uhrzeigersinn. Die Längsrichtung, bzw. die in Längsrichtung L verlaufende Komponente AK des Drahtelements 12x' bleibt gleich.

Ferner ist in den Fig. 2 und 3 zu erkennen, dass die inneren Drahtelemente 12a', 12b', 12c' und 12d' und die äußeren Drahtelemente 12a, 12b, 12c, 12d denselben Flechtwinkel aufweisen. Der Flechtwinkel ist derjenige Winkel eines Drahtelementes, den dieser zusammen mit der Längsachse einschließt. Das gilt für alle in der Anmeldung beschriebenen Flechtkonfigurationen.

Wie in Fig. 2 dargestellt, weist das Geflecht eine in Längsrichtung L des Hohlkörpers 10 vorne angeordnete Endmasche 18 bzw. eine vordere Endmasche 18 auf. Die Endmasche 18 weist in verschiedenen Axialrichtungen bezogen auf die Längsrichtung L des Hohlkörpers 10 angeordnete äußere Drahtelemente 12a, 12b, 12c, 12d auf. Die Drahtelemente 12a, 12b, 12c, 12d weisen somit im Bereich der vorderen Endmasche 18 eine Änderung der Axialrichtung auf.

Bei dem Ausführungsbeispiel gemäß Fig. 2 sind alle äußeren Drahtelemente 12a, 12b, 12c, 12d in der vorderen Endmasche 18 zusammengeführt, weshalb die Abschlusskante 16 im Bereich der vorderen Endmasche den größten Durchmesser hat. Die Anzahl der äußeren Drahtelemente 12a, 12b, 12c, 12d des ersten Abschnitts 16a und des zweiten Abschnitts 16b nimmt jeweils zu einem in Längsrichtung L des Hohlkörpers 10 vorderen Bereich 16c der Abschlusskante 16 zu und ist im vorderen Bereich 16c, konkret im Bereich der vorderen Endmasche 18 maximal.

Die äußeren Drahtelemente 12a, 12b, 12c, 12d können Teil einer einzigen Gruppe aus Drahtelementen sein, die im Bereich der vorderen Endmasche umgelenkt ist. Die Enden der jeweiligen Drahtelemente sind in diesem Fall in einem anderen Bereich des Geflechts fixiert, insbesondere am anderen Geflechtende. Alternativ können der von der einen Umfangsrichtung kommenden Drahtelemente 12a, 12b, 12c, 12d eine erste Gruppe und die aus der anderen Umfangsrichtung kommenden Drahtelemente 12a, 12b, 12c, 12d eine zweite Gruppe von Drahtelementen bilden. Die beiden Gruppen von Drahtelementen sind im Bereich der Spitze der vorderen Endmasche 18 miteinander verbunden.

Besonders in dem Fall, dass dieselben Drähte 12a, 12b, 12c, 12d als gesamte Gruppe umgelenkt sind, sind diese im Bereich der vorderen Endmasche 18 nicht spitz verformt, wie in den Zeichnungen schematisch dargestellt. Vielmehr weist das Ende der Masche eine Rundung auf. Wenn unterschiedliche Drahtgruppen zusammengeführt sind, können die Gruppen in distaler Richtung tangential zu einander verlaufen.

Bei dem Ausführungsbeispiel gemäß den Fig. 1 bis 3 muss der letzte Draht einer (abgewickelten) Seite, der vereinzelt ist, nicht mehr umgelenkt werden. Er verläuft viel mehr gerade weiter und wird zum inneren (geflechtbildenden) Drahtelement. Der letzte Draht kann auch ein Drahtbündel aus mehreren Drähten sein. Es ist auch möglich, dass an jeder Schlaufe jeweils zwei Drähte sich abzweigen. Es können auch mehr als zwei Drähte abgezweigt sein. Dabei entsteht eine Konfiguration, in welcher die Drähte in den verschiedenen Abschnitten, z.B. 8 Drähte, 6 Drähte, 4 Drähte, 2 Drähte verdrillt sind. Andere Konfigurationen sind ebenfalls möglich.

Im Bereich der Kontur der Abschlusskante 16, sind die Drähte verdrillt, wodurch eine kompakte Konfiguration entsteht. Dies ermöglicht das Einführen der Vorrichtung in kleine Katheterlumen. Die Abzweigung (bzw. Zufuhr) jedes einzelnen Drahtelementes kann so erfolgen, dass das Drahtelement sanft in die Verdrillung übergeht. Dies ist beispielsweise durch die Auslegung der Verdrillung und die genaue Position der Abzweigung möglich. Es ist auch möglich, dass die Drahtelemente nicht verdrillt sondern geflochten sind. Dies erhöht die Stabilität der Drahtstränge im Bereich der Abschlusskante 16. Eine Kombination aus Verdrillung und Flechten ist auch möglich.

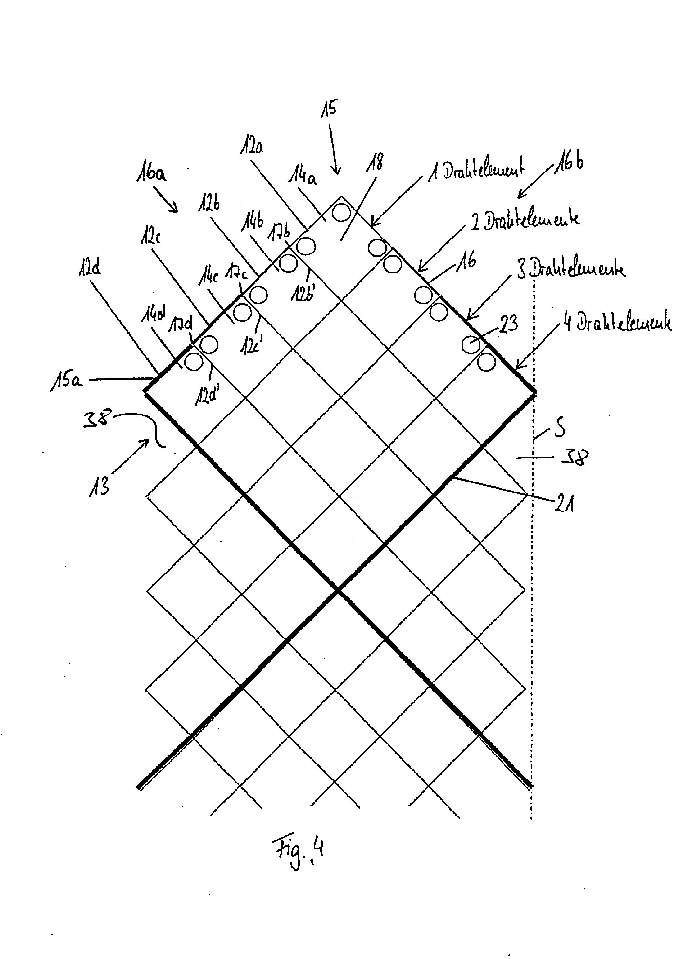

Ein weiteres Ausführungsbeispiel für eine Vorrichtung mit einer anderen Flechtkonfiguration ist in Fig. 4 dargestellt. Im Prinzip unterscheidet sich die Flechtkonfiguration gemäß Fig. 4 von der Flechtkonfiguration gemäß Fig. 1 bis 3 dadurch, dass sich beim Übergang der äußeren Drahtelemente 12a, 12b, 12c, 12d in die inneren Drahtelemente 12a', 12b', 12c' und 12d' die Richtung der Axialkomponente AK der Drähte ändert. Die Windungsrichtung, bzw. die Umfangskomponente UK bleibt beim Übergang dieselbe. Dieser Zusammenhang ist in Fig. 3b dargestellt. In Fig. 3b ist zu erkennen, dass die Axialkomponente AK des geflechtbildenden Drahtelementes 12x' in entgegengesetzte Richtung verläuft, wie die Axialkomponente AK des kantenbildenden Drahtelementes 12x.

Bei der Flechtkombination gemäß Fig. 4 nimmt die Anzahl der äußeren Drahtelemente 12a, 12b, 12c, 12d zur Spitze bzw. zur vorderen Endmasche 18 hin ab und ist dort minimal, im Gegensatz zu der Konfiguration gemäß Fig. 1 bis 3, bei der die Anzahl der äußeren Drahtelemente zur Spitze hin zunimmt. Im Übrigen ist das Aufbauprinzip dasselbe, wie bei dem Beispiel gemäß Fig. 1 bis 3. Auch bei dem Ausführungsbeispiel gemäß Fig. 4 sind gestaffelte Schlaufen vorgesehen, die die Endmaschen 14a, 14b, 14c, 14d bilden und die an verschiedenen Stellen 14a, 14b, 14c, 14d zusammengeführt sind, die entlang der Abschlusskante 16 einander nachgeordnet sind. Am Übergang von einer Schlaufe zur nächsten Schlaufe wird ein Draht der Drahtgruppe umgelenkt und zwar in die entgegengesetzte Axialrichtung (Fig. 3b).

Die letzte Schlaufe besteht aus einem Einzeldrahtelement 12a und ist im Bereich der vorderen Endmasche 18 angeordnet. Ferner ist in Fig. 4 zu erkennen, dass die letzte Drahtgruppe, die alle äußeren Drahtelemente, insbesondere die vier Drahtelemente des Beispiels gemäß Fig. 4 umfasst, im Geflecht fortgeführt ist. Dieser von der vorderen Endmasche 18 entfernt angeordnete Drahtstrang 21 ist in das Geflecht integriert und führt dazu, dass das Geflecht auf seiner ganzen Länge stabilisiert ist. Besonders vorteilhaft ist es, wenn die Drahtelemente des Drahtstranges verdrillt sind, insbesondere in der gleichen Windungsrichtung. Andere Drahtkombinationen, insbesondere eine andere Anzahl von Drähten des Drahtstranges ist möglich.

In den Fig. 6a, 6b sind zwei weitere Ausführungsbeispiele der erfindungsgemäßen Vorrichtung dargestellt, bei denen die äußeren Drahtelemente 12a, 12b, 12c, 12d der vorderen Endmasche 18 eine Verlängerung 19 bilden, die sich über die Kontur der Abschlusskante 16 hinaus erstreckt. Die Verlängerung 19 weist ein Funktionselement, insbesondere ein Verbindungselement 20 auf, das beispielsweise als Schlaufe ausgebildet ist. Die Schlaufe kann dazu benutzt werden, um die Vorrichtung mit einem Betätigungselement, insbesondere mit einem Führungsdraht eines Zufuhrsystems zu verbinden. Im Übrigen entspricht die Flechtkonfiguration der Ausführungsbeispiele gemäß den Fig. 6a, 6b derjenigen der vorstehend beschriebenen Ausführungsbeispiele gemäß den Fig. 1 bis 3 und 5.

Generell können die Drähte im Bereich der Verlängerung 19, insbesondere die äußeren Drahtelemente 12a, 12b, 12c, 12d miteinander verdrillt sein unabhängig davon, ob die Drähte in der Abschlusskante 16 verdrillt oder lose angeordnet sind. Die verdrillte Anordnung der Drahtelemente im Bereich der Verlängerung 19 wird also im Zusammenhang mit allen Drahtkonfigurationen, insbesondere mit verdrillten, losen, geflochtenen, mechanisch verbundenen oder stoffschlüssig verbundenen Drahtkonfigurationen offenbart. Die Verdrillung im Bereich der Verlängerung 19 erhöht die Stabilität. Anstelle der Schlaufe können die Drähte freie Enden aufweisen. Auf der Verdrillung im Bereich der Verlängerung 19 kann eine Hülse bzw. allgemein ein profiliertes Endstück befestigt sein. Die Hülse.bzw. das profilierte Endstück können aus einem röntgensichtbaren Material, beispielsweise aus Platin hergestellt sein. Die Verbindung zwischen der Hülse bzw. dem profilierten Endstück und den Drähten im Bereich der Verlängerung 19 kann in Form einer Schweißverbindung, einer Crimpverbindung oder einer Klebverbindung oder einer anderweitigen mechanischen Verbindung erfolgen. Beispielsweise können die von der Hülse umgebenen Drahtenden zusammen mit der Hülse stirnflächig verschweißt sein, insbesondere durch eine halbkugelförmige Schweißstelle. Die Stirnflächen der freien Enden können auch ohne Hülse stirnflächig verschweißt sein.

Vorzugsweise erstreckt sich die Verlängerung 19 parallel zur Längsachse des Geflechtes. Dies ist nicht zwingend. Die gerade Verlängerung kann radial nach außen vorstehen. Eine radial nach außen abgerundete Form der Verlängerung 19 ist ebenfalls möglich. Vorzugsweise erstreckt sich die Verlängerung 19 in derselben Ebene in der die schräge Abschlusskante 16 angeordnet ist. Die Ebene entspricht in der Regel einer gekrümmten und schrägen Schnittfläche durch einen kreiszylindrischen Hohlkörper. Dadurch wird ein sanfter Übergang zwischen der Abschlusskante 16 und der Verlängerung 19 gebildet. Die Zylindermantelfläche kann nach außen gewölbt sein (flaring).

Bei allen offenbarten Ausführungsbeispielen kann das Geflecht mindestens 8, 12, 16, 24, 32, 36, 40, 48, 60, 72, 84, 96 Drähte aufweisen, wobei dies der Anzahl der Drähte, die eine zu der Geflechtachse senkrechte Ebene schneiden, entspricht. Bei geschlossenen Schlaufen ist der tatsächliche Drahtanzahl halbiert. Der Flechtwinkel, insbesondere der Flechtwinkel der Abschlusskante, beträgt mindestens 20° 30° 40° 45° 50° 60° 70° 80°. Dadurch wird der ovale Bereich verkürzt. Der Flechtwinkel, insbesondere der Flechtwinkel der Abschlusskante, beträgt höchstens 80° 70° 60° 50° 45° 40° 30° 20°. Durch die damit erreichte sanfte Schräge wird die Einführung in den Katheter vereinfacht.

Das System ist in einen Katheter mit einem Innendurchmesser von höchstens 2 mm, insbesondere höchstens 1,8 mm, insbesondere höchstens 1,5 mm, insbesondere höchstens 1,3 mm, insbesondere höchstens 1,1 mm, insbesondere höchstens 1,0 mm, insbesondere höchstens 0,9 mm, insbesondere höchstens 0,8 mm, insbesondere höchstens 0,7 mm, insbesondere höchstens 0,6 mm, insbesondere höchstens 0,5 mm, insbesondere höchstens 0,4 mm, insbesondere höchstens 0,3 mm, insbesondere höchstens 0,2 mm einführbar.

Das Geflecht kann Drähte mit unterschiedlicher Wandstärke aufweisen.

Das Geflecht kann bespannt, bevorzugt mit PU, sein. Es kann teilweise bespannt sein. Der Korb zur Entfernung eines Thrombus kann zum Beispiel nur im distalen Bereich bespannt sein.

Die Drähte können aus Nitinol, Cobalt-Crom-Legierung oder Nitinoldrähten mit Platinkern hergestellt sein.

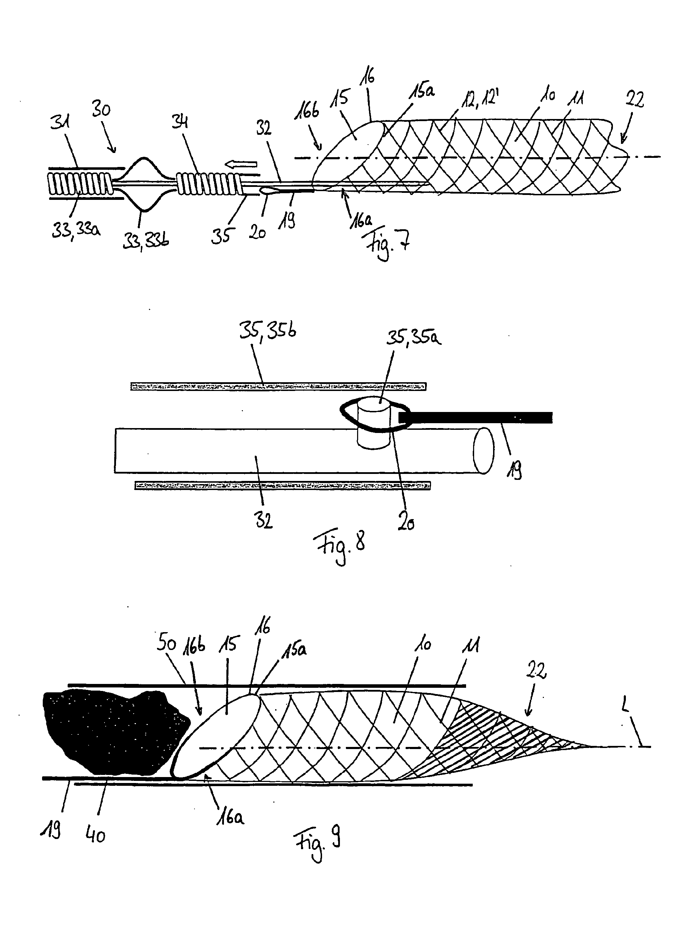

Die Kopplung der Vorrichtung gemäß Fig. 6a und 6b mit dem Führungsdraht eines Katheters ist in den Fig. 7 und 8 dargestellt. Das Zufuhrsystem ist allgemein mit dem Bezugszeichen 30 versehen und weist einen Katheter 31 auf, in dem ein Betätigungselement, insbesondere ein Führungsdraht 32 in Längsrichtung angeordnet ist. Der Führungsdraht 32 umfasst einen Abkopplungsmechanismus 33, der ein proximales Ende 33a aufweist. Das proximale Ende 33a des Abkopplungsmechanismus 33 ist fest mit dem Führungsdraht 32 verbunden. In distaler Richtung nachgeordnet ist ein Auslöseelement 33b vorgesehen, das mit dem proximalen Ende 33a verbunden ist und in radialer Richtung im Katheter 31 vorgespannt ist. Durch Freisetzen des Auslöseelements 33b, wie in Fig. 7 dargestellt, entspannt sich dieses in radialer Richtung, wodurch eine Verkürzung des Abkopplungsmechanismus 33 in längsaxialer Richtung bewirkt wird. Das Auslöseelement 33b ist mit dem Zwischenstück 34 verbunden, insbesondere mit einem auf dem Führungsdraht 32 längsverschieblich angeordneten Zwischenstück 34. Das Zwischenstück 34 ist mit einem Haltemittel 35, beispielsweise einer Hülse fest verbunden. Das Zwischenstück 34 und die Hülse 35 sind durch eine Bewegung des Auslöseelements 33b betätigbar, insbesondere in Längsrichtung entlang des Führungsdrahtes 32 zurückziehbar.

Das Haltemittel 35 umfasst konkret, wie in Fig. 8 dargestellt, eine Hülse 35b, die längsverschieblich entlang des Führungsdrahtes angeordnet ist, sowie einen Stift 35a bzw. ein Arretierungselement, der fest mit dem Führungsdraht 32 verbunden ist. Die Schlaufe 20 der Verlängerung 19 des Korbes umgreift den Stift 35a. Durch Zurückziehen der Hülse 35b wird die Arretierung des Stiftes 35a gelöst bzw. wird dieser freigegeben, so dass die Schlaufe 20 durch die Radialexpansion des Korbes radial nach außen bewegt wird. Damit wird der Korb vom Zufuhrsystem abgekoppelt.

Das Zufuhrsystem ist näher in der auf die Anmelderin zurückgehenden Anmeldung mit dem Titel "Zufuhrsystem für ein medizinisches Funktionselement", die am selben Tage eingereicht wurde, näher beschrieben. Der Inhalt dieser Anmeldung wird vollumfänglich in die vorliegende durch Verweis aufgenommen, da das Beispiel gemäß Fig. 6a, 6b mit dem darin offenbarten Zufuhrsystem kombinierbar ist.

In Fig. 9 ist ein Beispiel für einen möglichen Einsatz des erfindungsgemäßen Korbes bzw. allgemein für die erfindungsgemäße Vorrichtung dargestellt. In Fig. 9 ist zu sehen, dass die erfindungsgemäße Vorrichtung in einem Blutgefäß 50 zum Entfernen eines Thrombus bzw. Konkrements 40 angeordnet ist. Durch die Verlängerung 19 kann die glatte Abschlusskante 16, nachdem der Thrombus in den Korb eingezogen wurde, leicht in den Katheter zurückgezogen werden.

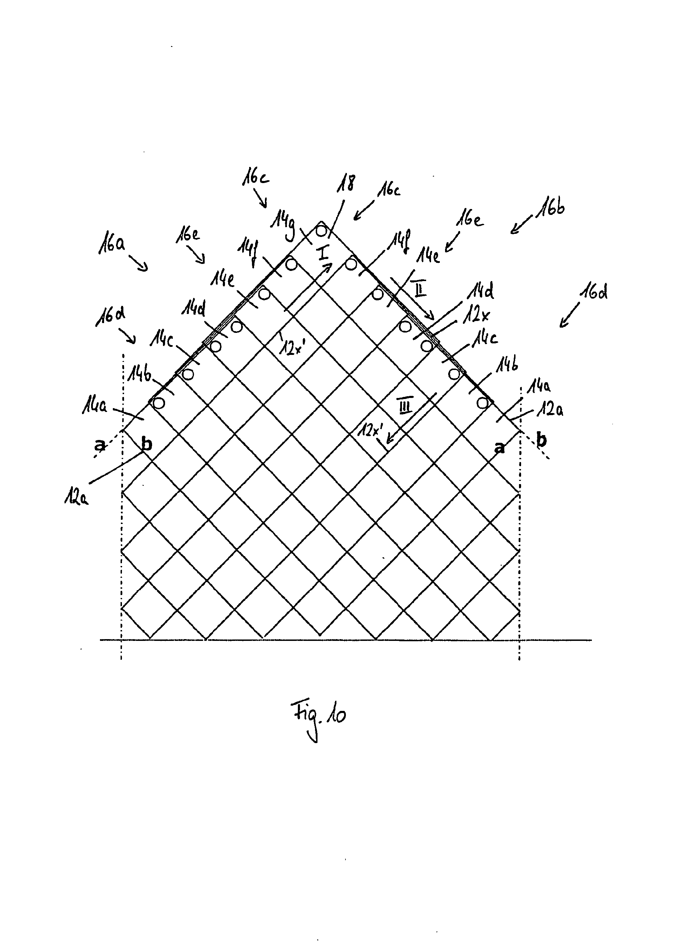

Weitere Ausführungsbeispiele mit unterschiedlichen Flechtkonfigurationen sind in den Figuren 10 bis 13 gezeigt, bei denen die äußeren Drahtelemente 12a bis 12g bzw. 12a bis 12d jeweils - in Umlaufsrichtung ULR des Randes 15a gesehen - unterschiedliche, d.h. in verschiedene Richtungen weisende Axialkomponenten AK1 und AK2 aufweisen und zwar in den beiden Abschnitten 16a, 16b der Abschlusskante 16.

Bei dem Ausführungsbeispiel gemäß Fig. 10 weist der erste Abschnitt 16a und der zweite Abschnitt 16b der Abschlusskante 16 jeweils einen vorderen Bereich 16c, einen hinteren Bereich 16d und einen dazwischen angeordneten Zwischenbereich 16e auf. Der vordere Bereich 16c befindet sich, wie in Fig. 10 dargestellt, in Längsrichtung L des Hohlkörpers vorne, d.h. in einem proximalen Bereich der Abschlusskante 16. Der hintere Bereich 16d befindet sich in einem distal gelegenen Bereich der Abschlusskante 16. Bei geneigt angeordneter Abschlusskante 16 grenzt an den hinteren Bereich 16d die geschlossene, insbesondere zylindrische Wandung des Geflechts an. Der Zwischenbereich 16e befindet sich zwischen dem vorderen und dem hinteren Bereich 16c, 16d. Ausgehend vom Zwischenbereich 16e nimmt die Anzahl der äußeren Drahtelemente 12a, 12b, 12c, 12d jeweils zum vorderen und hinteren Bereich 16c, 16d hin ab. Im vorderen und hinteren Bereich 16c, 16d ist die Anzahl der äußeren Drahtelemente 12a, 12b, 12c, 12d minimal. Im Zwischenbereich ist die Anzahl der äußeren Drahtelemente 12a, 12b, 12c, 12d maximal. Im vorliegenden Ausführungsbeispiel gemäß Fig. 10 ist der Zwischenbereich 16e symmetrisch zwischen dem vorderen und hinteren Bereich 16c, 16d angeordnet. Es ist auch möglich, den Zwischenbereich 16e näher am vorderen Bereich 16c bzw. näher am hinteren Bereich 16d anzuordnen.

Die Drahtführung einer Schlaufe ist in Fig. 10 anhand von Pfeilen dargestellt. Der Pfeil I bezeichnet den Verlauf eines geflechtbildenden Drahtelements 12x', das in die Abschlusskante 16 umgelenkt wird und entlang der Abschlusskante 16 in Pfeilrichtung II sich erstreckt. Dort geht das geflechtbildende Drahtelement 12x' in das kantenbildende Drahtelement 12x bzw. das äußere Drahtelement über. Im Abstand von vier Maschen vom geflechtbildenden Drahtelement 12x' (Pfeil I) wird das kantenbildende Drahtelement 12x wieder in das Geflecht zurückgelenkt und geht wieder in ein geflechtbildendes Drahtelement 12x' über, das mit dem Pfeil III bezeichnet ist. Aus den Pfeilen I, II, III ergibt sich der Verlauf der Schlaufe. Die weiteren Schlaufen sind jeweils um eine Endmasche versetzt entlang der Abschlusskante 16 angeordnet und entsprechen im Übrigen dem Verlauf der vorstehend beschriebenen Schlaufe. Daraus ergibt sich die Position des Zwischenbereichs 16e, bei dem die Anzahl der äußeren Drahtelemente 12a, 12b, 12c, 12d maximal ist. Der Grund hierfür liegt darin, dass im Zwischenbereich 16e sich alle Schlaufen überlappen.

Die Schlaufen des ersten Abschnittes 16a sind entsprechend angeordnet.

In Fig. 10 ist ferner zu erkennen, dass das äußere Drahtelement 12a der am weitesten von der vorderen Endmasche 18 entfernten Endmasche 14a nicht umgelenkt ist, sondern direkt in das Geflecht übergeht. Dies ist durch die Drahtbezeichnungen a, b in Fig. 10 verdeutlicht. Wenn die Abwicklung gemäß Fig. 10 zu einem zylindrischen Hohlkörper geschlossen ist, liegt die gestrichelte Linie b (rechte Seite) auf der durchgezogenen Linie b (linke Seite der Abwicklung).

Dies gilt für alle Ausführungsbeispiele dieser Anmeldung.

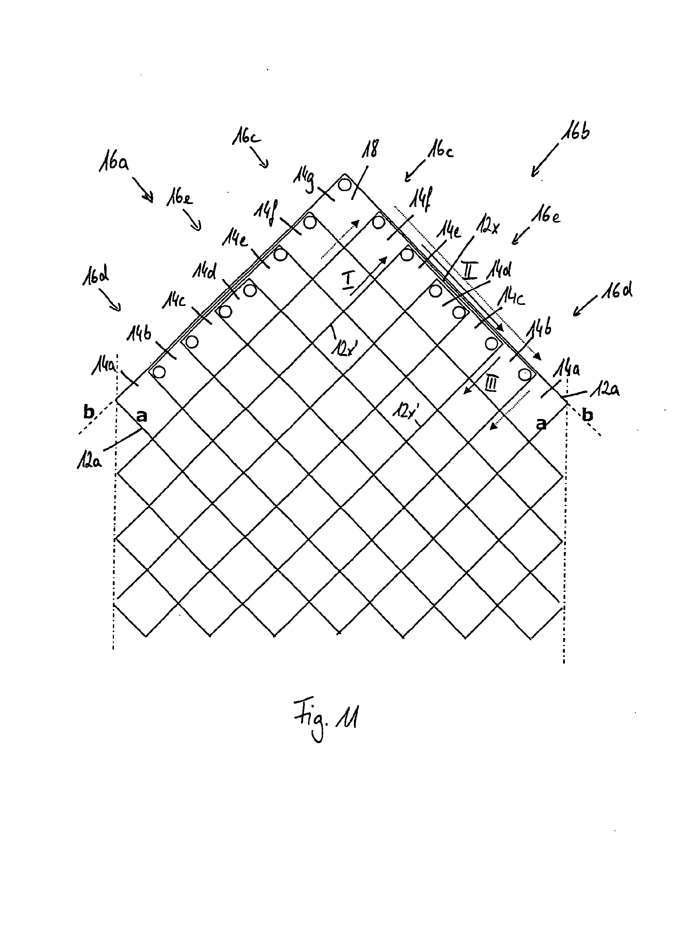

Das Ausführungsbeispiel gemäß Fig. 11 ist ähnlich aufgebaut wie das Ausführungsbeispiel gemäß Fig. 10 und umfasst mehrere ineinander verschachtelte Schlaufen, wie durch die punktierten und durchgezogenen Pfeile verdeutlicht. Wie in Fig. 11 dargestellt, sind die Schlaufen des ersten Abschnitts 16a bzw. die Schlaufen des zweiten Abschnitts 16b von außen nach innen zunehmend kleiner. Die äußere Schlaufe (strichpunktierte Pfeile) umfasst beispielsweise fünf Endmaschen. Die in der äußeren Schlaufe angeordnete innere Schlaufe (durchgezogene Pfeile) umfasst drei Endmaschen. Die kleinste Schlaufe, die innerhalb der mittleren Schlaufe (durchgezogene Pfeile) angeordnet ist, umfasst eine Endmasche. Damit sind die Schlaufen jeweils um eine Endmasche auf beiden Seiten nach innen versetzt. Auch bei dieser Anordnung ergibt sich ein Zwischenbereich 16e, wie im Zusammenhang mit Fig. 10 ausführlich beschrieben.

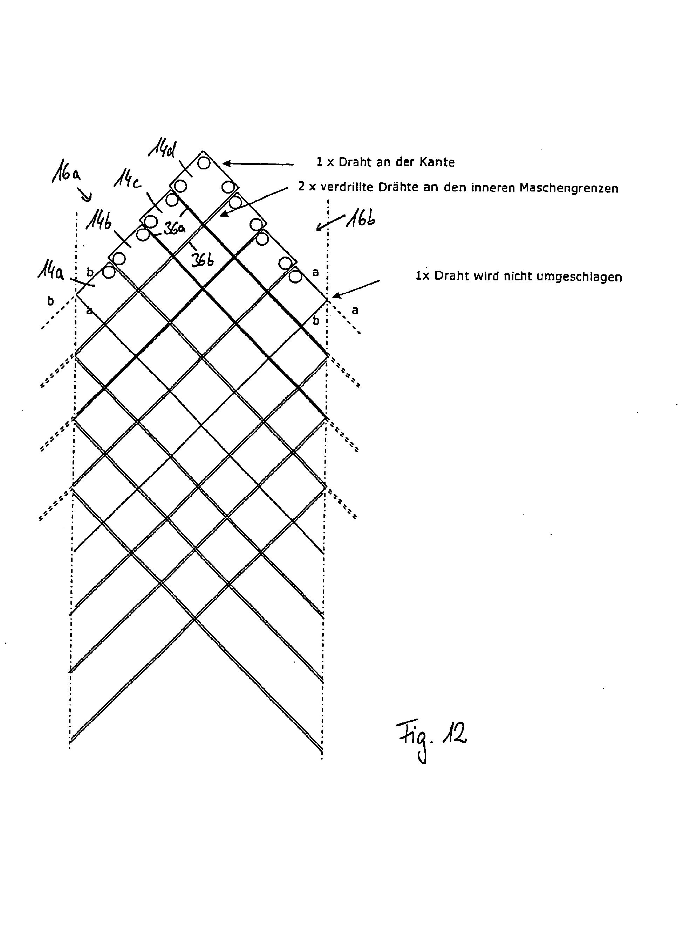

Ein anderes Ausführungsbeispiel ist in Fig. 12 dargestellt, bei dem die Abschlusskante 16 jeder Endmasche 14a, 14b, 14c, 14d nur einen Draht umfasst. Es ist auch möglich, dass jede Endmasche jeweils mehrere Drähte umfasst, wobei die Außendrähte 12a, 12b, 12c, 12d einander nicht überlappen, sondern jeweils auf die zugehörige Endmasche 14a, 14b, 14c, 14d begrenzt sind. Wie in Fig. 12 zu erkennen, bilden die inneren Drahtelemente 12a', 12b', 12c', 12d' innere Maschengrenzen 36a, 36b, die sich ausgehend von der Abschlusskante 16 ins Geflechtinnere erstrecken. Die inneren Maschengrenzen 36a schließen sich direkt an die Abschlusskante 16 bzw. die zugehörigen äußeren Drahtelemente 12a, 12b, 12c, 12d an. Die weitere innere Maschengrenze 36b verläuft parallel zur Abschlusskante 16 bzw. ist allgemein von dieser beabstandet und dieser gegenüber angeordnet. Zur Fixierung der Endmaschen 14a, 14b, 14c, 14d sind die inneren Maschengrenzen 36a und/oder 36b zumindest teilweise miteinander verbunden. Das bedeutet, dass die inneren Drahtelemente 12a, 12b, 12c, 12d im inneren Bereich der Maschen teilweise zusammengebunden sind, bevorzugterweise verdrillt sind. Beim Übergang der äußeren Drahtelemente 12a, 12b, 12c, 12d auf die inneren Drahtelemente 12a', 12b', 12c', 12d' kommt es mit einer Ausnahme immer zu einer Richtungsänderung des Drahtes in Umfangsrichtung. Lediglich bei dem Draht der letzten Endmasche, d.h. der Endmasche 14a, an die sich die geschlossene Wandung, insbesondere die zylindrische Wandung des Hohlkörpers anschließt, kommt es nicht zu einer Richtungsänderung des Drahtes in Umfangsrichtung. Der entsprechende Draht wird nicht umgeschlagen, sondern ins Geflecht fortgeführt, wie vorstehend im Zusammenhang mit Fig. 10 beschrieben. Die Ausführungsform gemäß Fig. 12 kann mit den vorstehend beschriebenen Ausführungen kombiniert werden. Das Ausführungsbeispiel gemäß Fig. 12 zeigt ferner, dass die einzelnen Endmaschen im Bereich der äußeren Drahtelemente 12a, 12b, 12c, 12d leicht zueinander versetzt sein können. Dabei sind die Endmaschen in Einzugsrichtung sukzessive weiter nach außen versetzt, so dass sich insgesamt eine nach innen zurückspringende Abschlusskante 16 ergibt, wie in Fig. 12 dargestellt, die ohne zu verkanten, in ein Zufuhrsystem eingezogen werden kann.

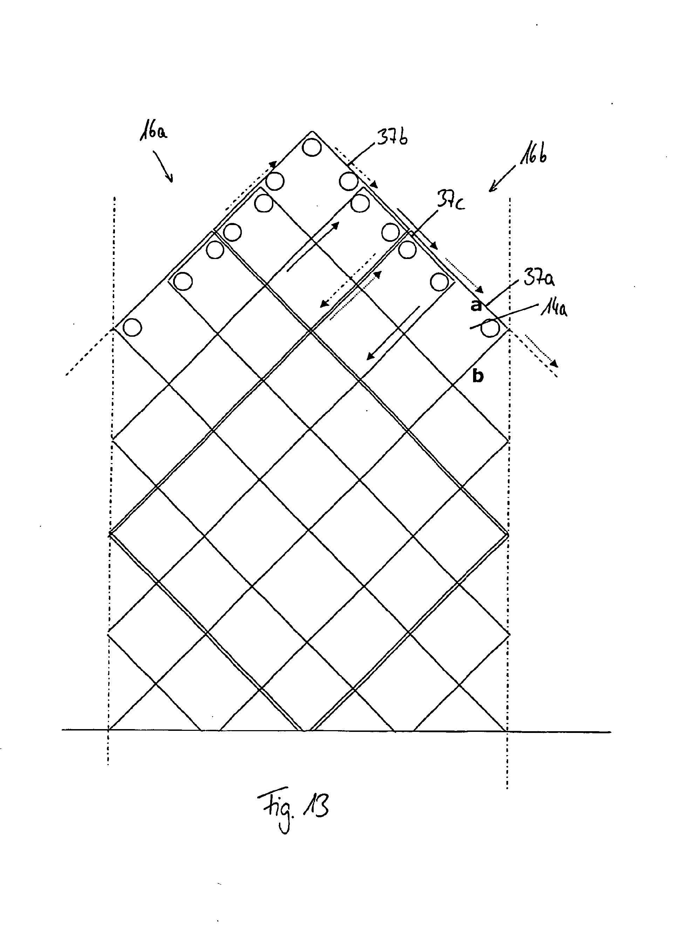

Bei dem Ausführungsbeispiel gemäß Fig. 13 sind die Beispiele gemäß den Figuren 11 und 12 miteinander kombiniert. Daraus ergibt sich eine Flechtkonfiguration, bei der die Endmaschen 14a, 14b, 14c, 14d des ersten Abschnitts 16a und des zweiten Abschnitts 16b jeweils wenigstens zwei Schlaufen 37a, 37b aufweisen, die entlang der Abschlusskante 16 nebeneinander angeordnet sind. Eine dritte Schlaufe 37c überlappt die beiden ersten und zweiten Schlaufen 37a, 37b. Der Drahtverlauf der ersten Schlaufe 37a ist durch die punktierten Pfeile dargestellt. In diesem Zusammenhang wird nochmals deutlich, dass das äußere Drahtelement der letzten Endmasche 14a nicht umgeschlagen wird, sondern im Geflecht fortgeführt wird. Die letzte Endmasche 14a grenzt direkt an die geschlossene Wandung bzw. die zylindrische Wandung des Geflechts an. Mit anderen Worten befindet sich die letzte Endmasche 14a im Scheitel der Abschlusskante 16. Die zweite angrenzende Schlaufe 37b ist durch die strichpunktierten Pfeile verdeutlicht, die den Drahtverlauf der zweiten Schlaufe 37b illustrieren. Aus dem Drahtverlauf wird klar, dass die erste und zweite Schlaufe einander nicht überlappen, sondern aneinander angrenzend entlang der Abschlusskante 16 angeordnet sind. Die beiden ersten und zweiten Schlaufen 37a, 37b sind von einer dritten Schlaufe 37c überlappt, deren Drahtverlauf durch die durchgezogenen Pfeile dargestellt ist. Die dritte Schlaufe 37c überlappt die beiden ersten und zweiten Schlaufen 37a, 37b jeweils um eine Endmasche. Andere Überlappungsgrade sind möglich. Die Überlappung kann, wie in Fig. 13, symmetrisch erfolgen. Es ist auch möglich, asymmetrische Überlappungen vorzusehen. Aus der Schlaufenüberlappung gemäß Fig. 13 ergibt sich, dass einige Drähte bzw. Drahtabschnitte innerhalb des Geflechts, also nicht im Bereich der Abschlusskante 16 verlaufen. Diese Drähte verlaufen parallel und können miteinander verbunden sein, beispielsweise durch Verdrillen oder andere Verbindungsarten. Andere Drahtabschnitte, insbesondere im Bereich der dritten Schlaufe 37c verlaufen im Bereich der Abschlusskante 16 zusammen und können ebenfalls verbunden sein oder lose angeordnet sein.

Damit ist klar, dass die vorstehend beschriebenen Ausführungsbeispiele unterschiedliche Kombinationen zulassen, die eine variable Auslegung des Geflechts ermöglichen, so dass die mechanischen Eigenschaften, wie beispielsweise die Radialkraft, Flexibilität und Feinmaschigkeit sehr präzise eingestellt werden können.

In allen Ausführungsbeispielen kann die Vorrichtung im expandierten Zustand am axialen Ende, vorzugsweise im Bereich der Abschlusskante 16, trichterförmig gestaltet (geflairt) sein. Das bedeutet, dass das Gittergeflecht in Richtung des axialen Endes aufgeweitet ist, also einen vorzugweise kontinuierlich steigenden Querschnittsdurchmesser aufweist. Bevorzugterweise ist die trichterförmige Aufweitung an einem distalen Ende des Gittergeflechts angeordnet. Das distale Ende entspricht dabei dem Axialende der Gitterstruktur, das bei der Freisetzung der Vorrichtung aus einem Katheter den Katheter bzw. die Katheterspitze zuerst verlässt. Das distale Ende ist also vom Anwender entfernt angeordnet, wogegen ein proximales Ende der Gitterstruktur bzw. der Vorrichtung dem Anwender zugewandt ist. Mit dem trichterförmig aufgeweiteten, distalen Ende der Gitterstruktur wird erreicht, dass die Expansion der Vorrichtung bei der Freilassung aus einem Zufuhrsystem erleichtert ist. Eine Aufweitung am distalen Axialende der medizinischen Vorrichtung ist insbesondere bei Rekanalisationsystemen oder Körben, insbesondere Thrombosefängern bzw. -filtern vorteilhaft, die sich im Hohlorgan distal aufspannen. Alternativ kann das proximale, also im Gebrauch anwendernahe Axialende des Gittergeflechts, eine trichterförmige, insbesondere geflairte, Struktur aufweisen. Diese Ausführungsform eignet sich für Vorrichtungen, die als Thrombosefänger bzw. Körbe mit einem proximal expandierbaren Axialende ausgebildet sind. Es ist ferner möglich, dass sowohl das proximale, als auch das distale Axialende des Gittergeflechts eine Trichterform aufweisen. Vorteilhaft ist diese Ausführungsform beispielsweise bei einer Vorrichtung, die als Dauerimplantat bzw. Stent, insbesondere Stenose-Stent oder Aneurysmen-Stent, ausgebildet ist. Im Allgemeinen ist es bevorzugt, wenn das trichterförmige Ende einen Zugang zu einem Hohlraum bildet, der innerhalb des Gittergeflechts ausgebildet ist. Ein weiteres Axialende der medizinischen Vorrichtung kann geschlossen sein oder ebenfalls eine Trichterform aufweisen.

Wie eingangs erläutert, sind die äußeren Drahtelemente 12a durch Umlenkung, d.h. durch Änderung der Windungsrichtung der inneren Drahtelemente 12a' gebildet. Ein äußeres und inneres Drahtelement 12a, 12a' bilden also ein einteiliges Drahtelement 12A, dessen freies Ende im Bereich der Spitze der Abschlusskante 16 bzw. am axialen Geflechtende 15 angeordnet und ggf. fixiert ist. Das innere Drahtelement 12a' bildet den geflechtbildenden Abschnitt des einteiligen Drahtelements 12A. Das äußere Drahtelement 12a ist durch Umlenkung des inneren Drahtelements 12a' gebildet und in die Abschlusskante 16 integriert. Das äußere Drahtelement 12a bildet den kantenbildenden Abschnitt des einteiligen Drahtelements 12A. Das innere Drahtelement 12a' entspricht also dem nicht-umgelenkten Drahtelement 12A und das äußere Drahtelement 12a dem umgelenkten Drahtelement 12A. Dies gilt für alle Drahtelemente, wobei das erste Drahtelement 12A, 12äußere Drahtelement 12a umfassen , die ohne Änderung der Spiralrichtung in die Abschlusskante 16 geführt sind. Bei Drähte, die nicht umgelenkt werden, entspricht 12a der Fortsetzung von 12a' in der Abschlusskante 16. Die folgenden Erläuterungen werden deshalb im Zusammenhang mit allen Ausführungsbeispielen bzw. allgemein im Zusammenhang mit der Erfindung offenbart.

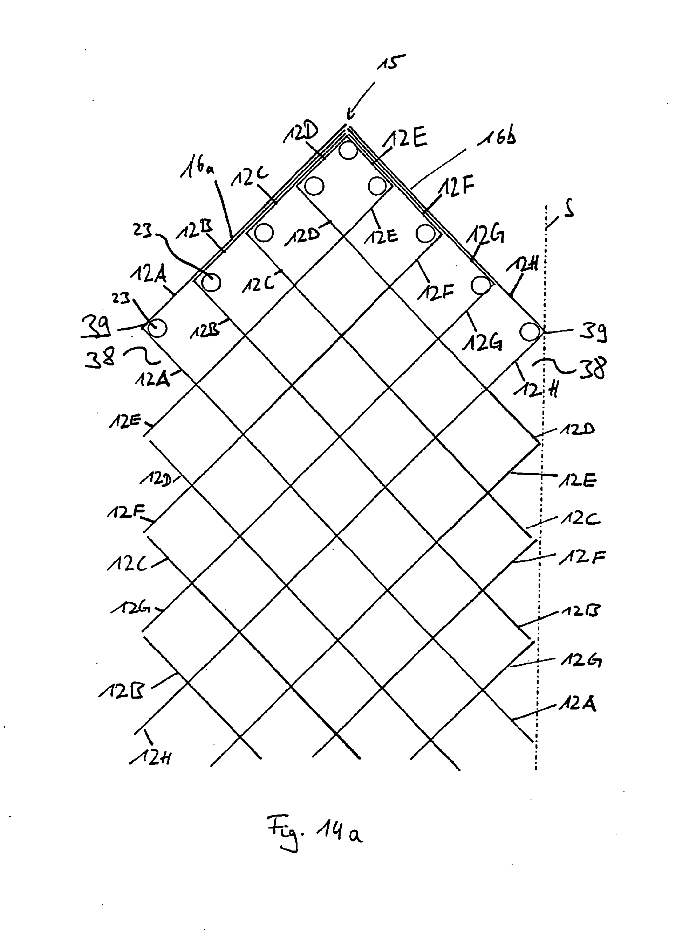

In den Figuren Fig. 14a, 14b, 15a, 15b und 16 ist eine medizinische Vorrichtung, insbesondere ein Implantat zum Entfernen von Konkrementen aus Körperhohlorganen, mit einem komprimierbaren und expandierbaren Gittergeflecht 11 gezeigt, das mehrere Drahtelemente 12A, 12B, 12C, 12D mit einer ersten Spiralrichtung und mehrere Drahtelemente 12E, 12F, 12G, 12H mit einer zweiten Spiralrichtung umfasst, die jeweils um eine gemeinsame Längsachse gewunden sind und sich zur Bildung von Maschen jeweils kreuzen. Das Gittergeflecht 11 weist eine umlaufende Abschlusskante 16 mit einem ersten und einem zweiten Abschnitt 16a, 16b auf, wobei sich der erste und zweite Abschnitt 16a, 16b in unterschiedlichen Spiralrichtungen entlang der Abschlusskante 16 erstrecken. Die beiden Abschnitte 16a, 16b treffen sich an der Spitze der Abschlusskante 16b bzw. am Geflechtende 15. Jeder der beiden Abschnitte 16a, 16b ist durch jeweils wenigstens zwei Drahtelemente gebildet, von denen jeweils wenigstens ein Drahtelemente beim Übergang vom Gittergeflecht 11 in die Abschlusskante 16 an einer Umlenkstelle umgelenkt ist derart, dass sich das umgelenkte Drahtelement entlang der Abschnittskante 16 in einer anderen Spiralrichtung als innerhalb des Gittergeflechts 11 erstreckt. Das umgelenkte Drahtelement erfährt also eine Änderung der Spiralrichtung beim Übergang vom Geflecht 11 in die Abschlusskante 16. Die Abschlusskante 16 ist dabei glatt und läuft kontinuierlich, also ohne Vorsprünge um.

Die Drahtelemente 12A, 12B, 12C, 12D mit der ersten Spiralrichtung sind axialsymmetrisch zu den Drahtelementen 12E, 12F, 12G, 12H mit der zweiten Spiralrichtung angeordnet, wie in Fig. 14a dargestellt. Die Ausführungen zu den Drahtelementen 12A, 12B, 12C, 12D mit der ersten Spiralrichtung werden auch im Zusammenhang mit den Drahtelementen 12E, 12F, 12G, 12H mit der zweiten Spiralrichtung offenbart. Das vom Geflechtende 15 bzw. der Spitze der Abschlusskante 16 am weitesten entfernte Drahtelement, das im Bereich des Scheitels 39 in die Abschlusskante 16 mündet, wird als erstes Drahtelement 12A bezeichnet. Das entsprechende Drahtelement mit der zweiten Spiralrichtung ist mit 12H bezeichnet. Das erste Drahtelement 12A ist am ersten Stift 23 umgelenkt und geht unter einer Änderung der Spiralrichtung in die Abschlusskante 16, konkret in den ersten Abschnitt 16a der Abschlusskante 16 über. Die Spiralrichtung des ersten Abschnitts 16a entspricht der zweiten Spiralrichtung der Drahtelemente 12E, 12F, 12G, 12H. Das erste Drahtelement 12A wechselt also beim Übergang vom Geflecht 11 in die Abschlusskante 16 von der ersten Spiralrichtung in die zweite Spiralrichtung.

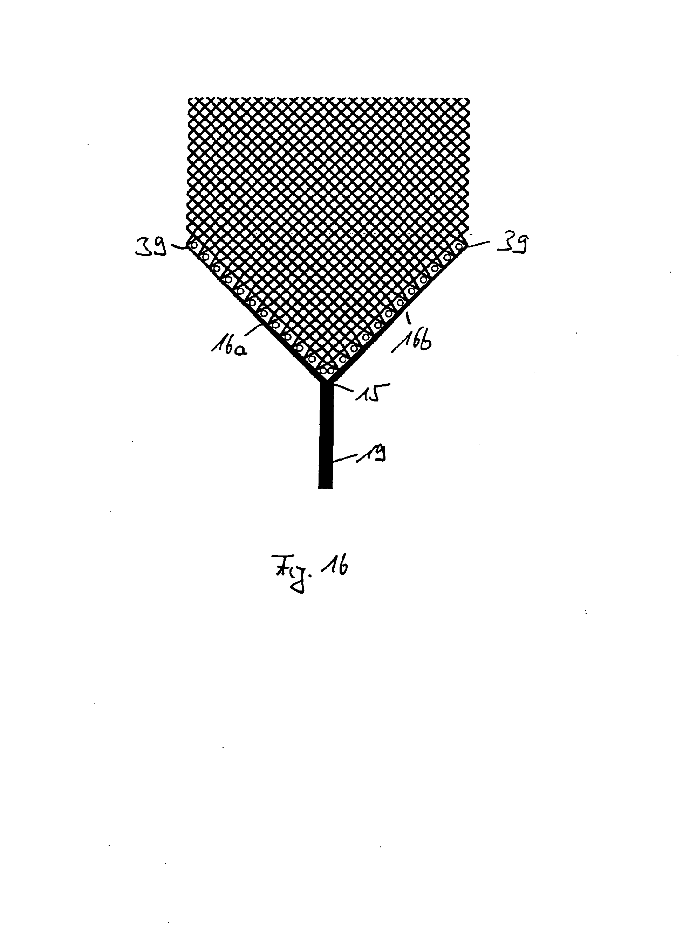

Ebenso wechselt das erste Drahtelement 12H mit der zweiten Spiralrichtung beim Übergang vom Geflecht 11 in die Abschlusskante 16, konkret beim Übergang in den zweiten Abschnitt 16b die Spiralrichtung derart, dass das erste Drahtelement 12H im Bereich der Abschlusskante 16 in der ersten Spiralrichtung verläuft. Bezogen auf dieselbe Umlaufrichtung ULR des Randes 15a (s. bspw. Fig. 2) weisen somit die beiden ersten Drahtelemente 12A, 12H entgegen gesetzte Axialkomponenten AK1, AK2 auf, wie im Zusammenhang mit Fig. 2, 3, 3a, 3b näher beschrieben. Dies gilt für alle Drahtelemente, die in die Abschlusskante 16 übergehen, insbesondere für die zweiten Drahtelemente 12B, 12G, die dritten Drahtelemente 12C, 12F und die vierten Drahtelemente 12D, 12E sowie für etwaige weitere Drahtelemente, die in den ersten und zweiten Abschnitt 16a, 16b integriert sind, wie in Fig. 16 dargestellt.

Aufgrund der aufgeschnittenen Darstellung gemäß Fig. 14a endet bspw. das letzte Drahtelement 12E mit der zweiten Spiralrichtung, das nahe der Spitze der Abschlusskante 16 bzw. des Geflechtendes 15 in den zweiten Abschnitt 16b der Abschlusskante 16 mündet, an der Schnittlinie S. In der nichtgeschnittenen dreidimensionalen Form der Vorrichtung setzt dagegen das letzte Drahtelement 12E sein Spiralform auf der gegenüberliegenden Seite der Darstellung gemäß Fig. 14a in distaler Richtung, also in der Zeichnungsebene nach unten, fort, wie durch den Verlauf des Drahtelements 12E gezeigt. Dies gilt für alle Drahtelemente 12A, 12B, 12C, 12D, 12E, 12F, 12G, 12H entsprechend.

Die Drahtelemente 12A, 12B, 12C, 12D, 12E, 12F, 12G, 12H münden aus dem Geflecht 11 kommend auf unterschiedlichen Höhen in die Abschlusskante 16 jeweils unter demselben Winkel. Dabei mündet ein Teil, insbesondere die Hälfte der Drahtelemente 12A, 12B, 12C, 12D in den ersten Abschnitt 16a der Abschlusskante 16 und ein Teil, insbesondere die andere Hälfte der Drahtelemente 12E, 12F, 12G, 12H in den zweiten Abschnitt 16b der Abschlusskante 16. Die beiden Abschnitte 16a, 16b bilden symmetrische Hälften bzw. allgemein Segmente der Abschlusskante 16.

Im Zusammenhang mit allen Ausführungsbeispielen sowie generell im Zusammenhang mit der Erfindung wird offenbart, dass die Drahtelemente 12A, 12B, 12C, 12D, 12E, 12F, 12G, 12H, die in die Abschlusskante 16 übergehen, bzw. die inneren Drahtelemente 12a', 12b', 12c', 12d' im Bereich der Abschlusskante 16 eine abrupte bzw. diskontinuierliche Änderung der Spiralrichtung bzw. der Umfangsrichtung erfahren. Die Drahtelemente 12A, 12B, 12C, 12D, 12E, 12F, 12G, 12H bilden also einen definierten Winkel beim Übergang in die Abschlusskante 16, bspw. einen Winkel von ca. 90°. Andere Winkel sind möglich, insbesondere in einem Bereich von 90° bis 60°, vorzugsweise 70°. Der Flechtwinkel kann von 45° bis 60°, insbesondere ca. 55° betragen. Bspw. korreliert der Flechtwinkel von 45° mit dem Übergangswinkel von 90°. Die anderen Winkel verhalten sich entsprechend.

Durch dieselbe Orientierung der Drahtelemente 12A, 12B, 12C, 12D, 12E, 12F, 12G, 12H beim Übergang in die Abschlusskante 16, also durch die Umlenkung unter demselben Winkel wird erreicht, dass alle Drahtelemente 12A, 12B, 12C, 12D, 12E, 12F, 12G, 12H dieselbe Länge aufweisen. Die Drahtelemente 12A, 12B, 12C, 12D, 12E, 12F, 12G, 12H münden also an verschiedenen diskreten Stellen unter demselben Winkel in die Abschlusskante 16. Bspw. verläuft das erste Drahtelement 12A entlang der gesamten Länge des ersten Abschnitts 16a. Das letzte Drahtelement 12D verläuft nur entlang einer Masche im Bereich der Abschlusskante 16, aber dafür entsprechend länger im Geflecht, so dass sich insgesamt dieselbe oder zumindest in etwa dieselbe Drahtlänge ergibt. Dasselbe gilt für die übrigen Drahtelemente.

Durch die im Wesentlichen selbe Länge der Drahtelemente 12A, 12B, 12C, 12D, 12E, 12F, 12G, 12H wird die Crimpbarkeit des Geflechts verbessert, da es zu keinen, zumindest aber zu keinen signifikanten Verzerrungen beim Komprimieren kommt.

Alle Drahtelemente 12A, 12B, 12C, 12D, 12E, 12F, 12G, 12H bzw. alle inneren Drahtelemente 12a', 12b', 12c', 12d' können diskontinuierlich in die Abschlusskante 16 umgelenkt sein. Dies gilt sowohl für innere Drahtelemente 12', die aus Einzeldrähten oder die jeweils aus Drahtbündeln gebildet sind. Es ist auch möglich, dass alle Drahtelemente 12B, 12C, 12D, 12E, 12F, 12G, bzw. alle inneren Drahtelemente 12b', 12c', 12d' bis auf die ersten Drahtelemente 12A, 12H bzw. bis auf die ersten inneren Drahtelemente 12a' vollständig umgelenkt sind.

Bei den ersten Drahtelementen 12A, 12H, die im Bereich des Scheitels 39 in die Abschlusskante münden, sind daher drei Möglichkeiten zu unterscheiden. Die beiden ersten Drahtelemente 12A, 12H sind im Bereich des Scheitels umgelenkt und ändern beim Eintritt in die Abschlusskante jeweils die Spiralrichtung, wie in Fig. 14a gezeigt. Durch die abrupte Änderung der Spiralrichtung entsteht im Scheitel 39 ein Spalt. Der Spalt entspricht einer offenen Zelle 38, die direkt an den Scheitel der Abschlusskante 16 angrenzt. Die beiden Hälften der Zelle 38 sind in der abgewickelten Darstellung gemäß Fig. 2 und Fig. 14a gezeigt. Die offene Zelle 38 behindert die Einziehbarkeit des Geflechtes nicht, da die Abschlusskante 16 bis auf den Scheitel kantenlos ausgebildet ist bzw. kantenlos und kontinuierlich umläuft. Diese Ausgestaltung der Erfindung ist vom Begriff "kantenlos" umfasst.

Die beiden ersten Drahtelemente 12A, 12H können jeweils aus Einzeldrähten oder aus Drahtbündeln mit mehreren Einzeldrähten bzw. aus einer entsprechenden Kombination bestehen.

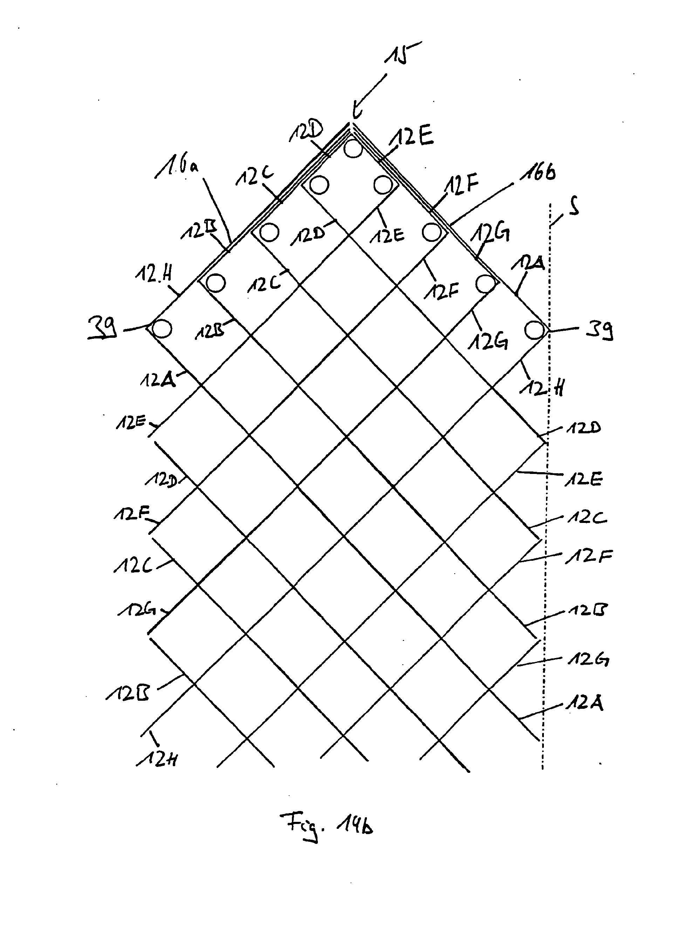

Alternativ können die beiden ersten Drahtelemente 12A, 12H im Bereich des Scheitels ihre Spiralrichtung beibehalten, also nicht umgelenkt sein, wie in Fig. 14b gezeigt. Das in der aufgeschnittenen Darstellung an der Schnittlinie S endende erste Drahtelement 12H setzt sich auf der gegenüberliegenden (linken) Seite des Geflechts fort und geht in die Abschlusskante 16 ohne Änderung der Spiral- bzw. Umfangsrichtung über. Damit überkreuzen sich die beiden ersten Drahtelemente 12A, 12H im Scheitel 39. Es wird kein Spalt gebildet. Die beiden ersten Drahtelemente 12A, 12H können auch hier jeweils aus Einzeldrähten oder aus Drahtbündeln mit mehreren Einzeldrähten bzw. aus einer entsprechenden Kombination bestehen.

Wenn die beiden ersten Drahtelemente 12A, 12H jeweils aus Drahtbündeln mit mehreren Einzeldrähten bestehen, kann ein Teil der Einzeldrähte eines Bündels umgelenkt sein. Der andere Teil kann die Spiralrichtung beibehalten. Damit sind die Einzeldrähte eines ersten Drahtelements 12A, 12H im Scheitel 39 aufgeteilt und gehen teilweise in den ersten Abschnitt 16a und teilweise in den zweiten Abschnitt 16b über. Auch hier wird kein Spalt gebildet. Dies gilt für beide erste Drahtelemente 12A, 12H. Im übrigen entsprechen sich die vorgenannten Ausführungsbeispiele.

Für die beiden ersten Drahtelemente 12A, 12H bestehen somit folgende Kombinationsmöglichkeiten: Alle Einzeldrähte werden jeweils umgelenkt oder werden jeweils ohne Richtungsänderung in die Abschlusskante 16 geführt. Alle Einzeldrähte des einen Drahtelements 12A werden umgelenkt und alle Einzeldrähte des anderen Drahtelements 12H werden nicht umgelenkt. Alle Drähte münden in denselben Abschnitt 16a, 16b. Alle Einzeldrähte des einen Drahtelements 12A werden umgelenkt oder werden nicht umgelenkt. Die Einzeldrähte des anderen Drahtelements 12H werden gesplittet und teilweise umgelenkt bzw. nicht-umgelenkt.

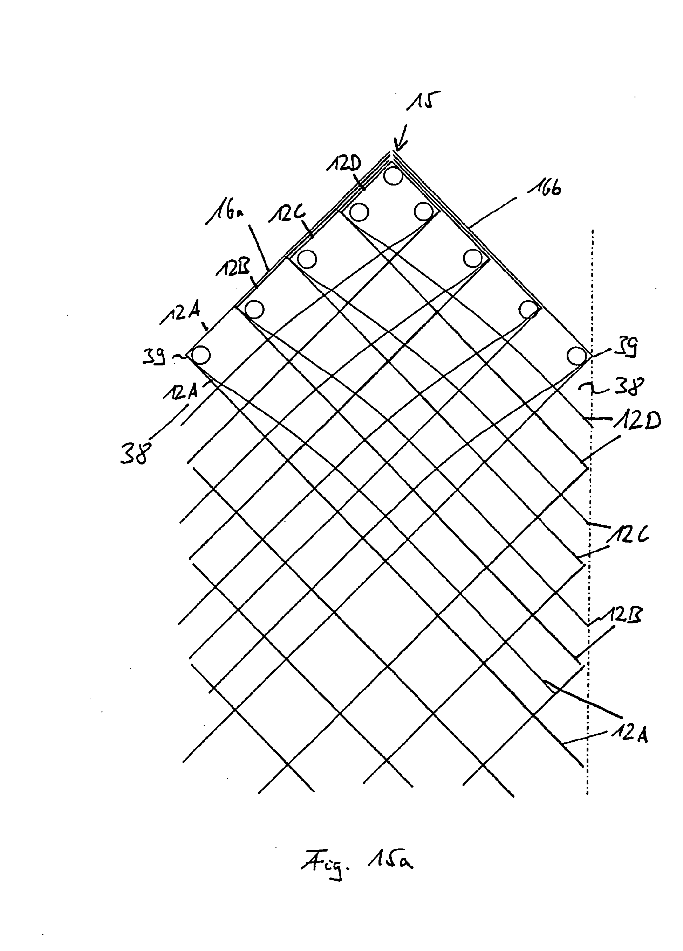

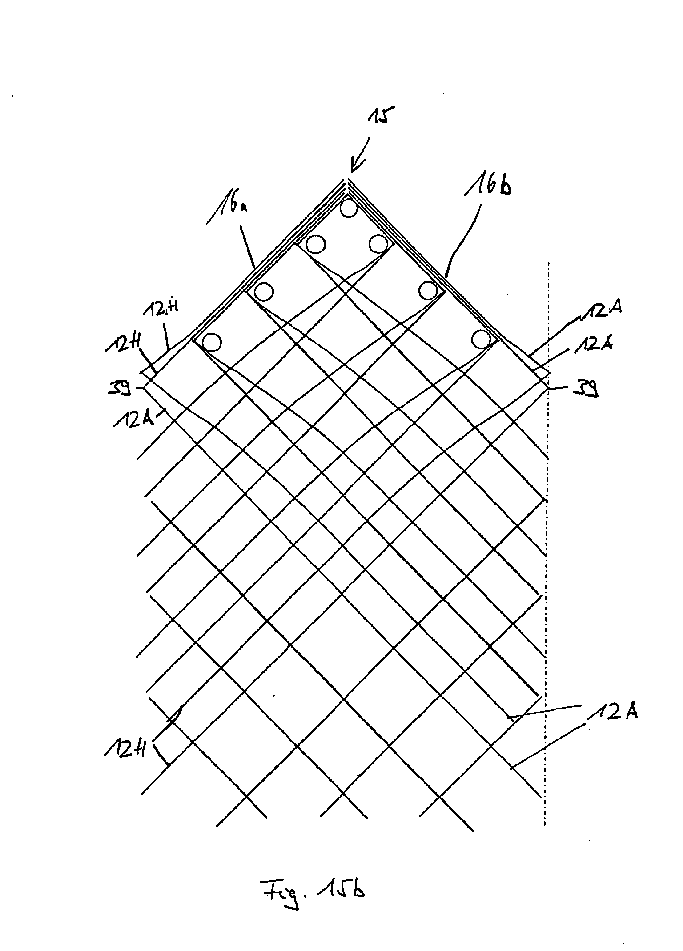

Die Ausführungsbeispiele gemäß Fig. 15a, 15b basieren auf den Ausführungsbeispielen gemäß Fig. 14a, 14b. Zusätzlich ist bei den Ausführungsbeispielen gemäß Fig. 15a, 15b vorgesehen, dass die Drahtelemente 12A, 12B, 12C, 12D, 12E, 12F, 12G, 12H jeweils Einzeldrähte umfassen. Die Einzeldrähte der jeweiligen Drahtelemente 12A, 12B, 12C, 12D, 12E, 12F, 12G, 12H verlaufen im Geflecht 11 seitlich beabstandet. An der Abschlusskante 16 kommen die Einzeldrähte der jeweiligen Drahtelemente 12A, 12B, 12C, 12D, 12E, 12F, 12G, 12H zusammen und sind gemeinsam in die Abschlusskante 16 umgelenkt. Die entlang der Abschlusskante 16 verteilt, insbesondere mit gleichem Abstand verteilt angeordneten Umlenkstellen sind durch die Umlenkstifte 23 definiert, die nicht zur Vorrichtung, sondern zum Herstellwerkzeug gehören. Die Anordnung der Umlenkstifte 23 bestimmt die Form der Abschlusskante 16.

Der Abstand zwischen den Einzeldrähten der jeweiligen Drahtelemente 12A, 12B, 12C, 12D, 12E, 12F, 12G, 12H verringert sich zumindest im Bereich der letzte Masche, insbesondere im Bereich der letzten beiden Maschen vor der Abschlusskante 16, bis die Einzeldrähte sich im Bereich der Umlenkstelle berühren und gemeinsam entlang der Abschlusskante 16 weiterlaufen. Bei der Verringerung des Abstands nähern sich die Einzeldrähte tangential. Der tangentiale Zusammenlauf ist auf die Einzeldrähte jeweils eines Drahtelements beschränkt. Die Umlenkung in die Abschlusskante 16 hinein erfolgt diskontinuierlich. Damit kommt es aufgrund des tangentialen Zusammenlaufs vor der Abschlusskante 16 nur zu einer vernachlässigbaren Verzerrung beim Crimpen. Bei dem Beispiel gemäß Fig. 15a sind wie beim Beispiel gemäß Fig. 14a die Einzeldrähte der beiden ersten Drahtelemente 12A, 12H umgelenkt, so dass sich ein Spalt im Bereich des Scheitels 39 bildet.

Die Verzerrung des Geflechts 11 wird auch dadurch begrenzt, dass die Anzahl der Einzeldrähte pro Drahtelement 12A, 12B, 12C, 12D, 12E, 12F, 12G, 12H beschränkt ist insbesondere auf maximal 6, insbesondere maximal 5, insbesondere maximal 4, insbesondere maximal 3, insbesondere maximal 2 Einzeldrähte.

An jedem Stift kommen zwei Drähte, die im Geflecht kurz vor dem Stift zusammenlaufen. Es sind aber nur jeweis zwei Drähte pro Stift. Die Verzerrung ist dadurch reduziert. Hauptsache ist, dass die Anzahl an Drähte, die tangential zusammenläuft, mit gradueller oder abrupter Änderung des Verlaufs, reduziert ist. Das Verhältnis der Anzahl der Einzeldrähte pro Drahtelement zur Gesamtanzahl der Drähte beträgt höchstens 25%, insbesondere höchstens 20%, insbesondere höchstens 15%, insbesondere höchstens 10%, insbesondere höchstens 8%, insbesondere höchstens 6%, insbesondere höchstens 5% insbesondere höchstens 4%.

Der Vorteil der Mehrfachdrahtführung besteht darin, dass bei einem sehr feinem Drahtgeflecht aufgrund der Vielzahl der Einzeldrähte eine entsprechend enge Positionierung der Stifte 23 des Herstellungsdorns durch den maximalen Stiftdurchmesser begrenzt ist. Durch die Bildung von Drahtbündeln kann die Abschlusskante 16 mit einer ausreichend großen Zahl von Umlenkstiften 23 gebildet werden, wobei die Feinmaschigkeit des Geflechts beibehalten wird. Die Einzeldrähte der Drahtbündelkönnen parallel, d.h. unverdrillt nebeneinander angeordnet sein. Die Einzeldrähte können auch verdrillt oder verflochten sein. Die parallel zueinander geführten Einzeldrähte der jeweiligen Drahtelemente 12A, 12B, 12C, 12D, 12E, 12F, 12G, 12H können lose Drahtbündel bilden. Unter einem losen Drahtbündel wird ein Drahtbündel verstanden, bei dem die Einzeldrähte nicht miteinander fixiert sind derart, dass diese zueinander relativbeweglich sind zumindest verglichen mit einer verdrillten oder geflochtenen Drahtkonfiguration, bei der die Relativbewegung der Einzeldrähte eingeschränkt ist.

Die Art der Drahtführung bis zum Stift 23 ist durch die Flechtart bestimmt. Diese kann bspw. 1 über 1, 1 über 2 oder ähnlich erfolgen und sorgt dafür, dass die Annäherung der Drähte erst kurz vor dem Stift erfolgt. Außerdem stabilisiert diese das Geflecht. Die Flechtart 2 über 2, 2 über 4, 4 über 4 oder ähnlich führt dazu, dass die Annäherung sanfter erfolgt bzw. der Annäherungsbereich länger ist. Die Flechtart kann sich ändern. Bspw. kann im Geflecht die Flechtart 1 über 1 und mit zunehmender Nähe zur Abschlusskante 16 die Flechtart 2 über 2, 2 über 4, 4 über 4 oder ähnliches vorliegen.

Bei dem Ausführungsbeispiel gemäß Fig. 15b sind die Einzeldrähte der beiden ersten Drahtelemente 12A, 12H nicht umgelenkt (vgl. Fig 14b), sondern münden ohne Wechsel der Spiralrichtung in den jeweiligen Kantenabschnitt 16a, 16b. Die Einzeldrähte überkreuzen sich daher im Bereich des Scheitels 39. Deshalb sind in diesem Bereich keine Umlenkstifte 23 eingezeichnet. Die Umlenkstifte können in diesem Bereich vorhanden sein. Die tangentiale Annäherung der Einzeldrähte der beiden ersten Drahtelemente 12A, 12H erfolgt u.a. entlang der Abschlusskante 16 und zwar in etwa über die Kantenlänge einer höchsten zwei Maschen. Im weiteren Verlauf der Abschlusskante 16 berühren sich die Einzeldrähte der beiden ersten Drahtelemente 12A, 12H. Bei einer Anzahl von 2 Einzeldrähten pro Drahtelement 12A, 12H ist die Verzerrung unproblematisch. Auch hier sind bis zu 6 Einzeldrähte insbesondere maximal 5, insbesondere maximal 4, insbesondere maximal 3, insbesondere maximal 2 Einzeldrähte möglich. Der tangentiale Zusammenlauf ist bspw. auf 2 Drähte beschränkt. Die Verzerrung ist damit ebenfalls beschränkt. Derjenige Draht, der einen gewissen Abstand zu der Abschlusskante 16 hat, ist überdies außerhalb der Abschlusskante angeordnet und gehört somit nicht zum Geflecht 11. Die Verzerrung ist auch aus diesem Grund weiter eingeschränkt. Die Abschlusskante 16 ist in Richtung Geflecht klar durch den Verlauf der Stifte definiert.

Ein weiteres Beispiel für eine Mehrfachdrahtführung mit wenigstens 2, insbesondere wenigstens 3, wenigstens 4 Drahtelementen mit jeweils mehreren Einzeldrähten, insbesondere mit zwei Einzeldrähten ist in Fig. 16 dargestellt. Alle Drahtelemente bzw. Einzeldrähte, die sich innerhalb des Bereiches zwischen Scheitel 39 und Spitze 15 befinden und Innendrähte darstellen, also nicht bereits zu der Abschlusskante 16 gehören, erfahren eine abrupte Änderung der Spiralrichtung in der Richtung des jeweiligen Abschlusskanteabschnitts 16a, 16b.

Alle Merkmale der Beispiele gemäß Fig. 14a, 14b, 15a, 15b und 16 werden im Zusammenhang mit allen übrigen Ausführungsbeispielen offenbart und beansprucht.

Zur Durchführung des Verfahren zur Herstellung einer Vorrichtung gemäß den Fig. 1 bis 9 wird ein Flechtdorn verwendet, auf dessen Umfang die in den Fig. 2, 3 und 4 dargestellten Stifte 23 angeordnet sind. Die Stifte 23 dienen dazu, die Windungsrichtung und/oder die Richtung der Axialkomponente der Drahtelemente beim Übergang von den geflechtbildenden auf die kantenbildenden Drahtelemente zu ändern. In Fig. 3 sind die Stifte 23 nur auf einer Seite der inneren Drahtelemente 12' eingezeichnet. Wie in Fig. 2 dargestellt sind in der Praxis auf beiden Seiten Stifte 23 vorgesehen. Der bezogen auf das umgelenkte Drahtelement 12' innen angeordnete Stift 23 ist, wie für den Fachmann ersichtlich, der für die Umlenkung des inneren Drahtelements 12' erforderliche Stift. Bei einer alternativen Anordnung der Stifte abweichend von der Anordnung gemäß Fig. 2 genügt es, wenn jeweils ein Stift 23 auf der Innenseite des umgelenkten inneren Drahtelements 12' angeordnet ist. Im Ausführungsbeispiel gemäß Fig. 3 sind dazu die eingezeichneten Stifte 23 jeweils auf die andere Seite (linke Seite in Fig. 3) der inneren Drahtelemente 12' zu versetzen.

Wenn bei dem Ausführungsbeispiel gemäß Fig. 2 alle inneren Drahtelemente 12', also auch das erste innere Drahtelement 12a' umgelenkt sind, ist ein weiterer Stift 23 jeweils in der ersten Endmasche 14a auf der Innenseite des umgelenkten ersten inneren Drahtelements 12a' angeordnet. Bei dem Ausführungsbeispiel gemäß Fig. 16 können weitere Umlenkstifte direkt im Bereich der Spitze und/oder seitlich von der Verlängerung 19 vorgesehen sein, die die Verdrillung der Verlängerung 19 begrenzen. Bei einer radialen Auslenkung der Spitze bzw. der Verlängerung 19 kann ein Dorn mit einer entsprechenden Ausweitung verwendet werden.

Die nachfolgende Beschreibung eines Herstellungsverfahrens für die erfindungsgemäße Vorrichtung gilt für alle Ausführungsbeispiele.

Im Allgemeinen wird zum Herstellen einer erfindungsgemäßen Vorrichtung, insbesondere nach einem der vorstehend erläuterten Ausführungsbeispiele, eine Flechtmaschine eingesetzt. Mit der Flechtmaschine werden die Drahtelemente automatisch verflochten, um das Geflecht 11 zu bilden. Die Flechtmaschine umfasst Spulen, auf die jeweils ein Drahtelement aufgewickelt ist. Die Spulen werden kreisförmig um einen gemeinsamen Flechtpunkt geführt und die Drahtelemente dabei von den Spulen abgewickelt. Während des Flechtvorgangs werden die Spulen ferner radial verschoben. Das bedeutet, dass die Spulen auf den Flechtpunkt hin oder von dem Flechtpunkt weg bewegt werden. Dazu sind die Spulen auf Spulenträgern bzw. Klöppeln angeordnet. Insbesondere können jeweils zwei Spulen auf einem Klöppel angeordnet sein. Vorzugsweise sind mehre Gruppen von Klöppeln vorgesehen, wobei wenigstens eine erste Gruppe von Klöppeln gegenläufig zu einer zweiten Gruppe von Klöppeln um den Flechtpunkt rotiert. Dabei umfahren sich die Klöppeln schlangenlinien- oder slalomartig. Die Rotationsrichtung der Klöppelgruppen bestimmt die Windungsrichtung der Drahtelemente im rohrförmigen Geflecht. An dem Axialende, das eine besonders glatte Kante aufweisen soll, werden die Drahtelemente entsprechend umgelenkt, indem die Rotationsrichtung der Klöppel umgekehrt wird. Um die Drahtelemente in der Abschlusskante zu verdrillen, werden jeweils zwei Spulen um eine gemeinsame Verdrillachse bzw. der die zwei Spulen tragende Klöppel um die Klöppelachse rotiert. Die Rotation der Spulen um die gemeinsame Verdrillachse kann mit der Rotation der Klöppelgruppen um die Flechtachse bzw. den Flechtpunkt überlagert sein.

Mit den an sich bekannten Flechtmaschinen sind sowohl schlauchartige bzw. rohrförmige Gitterstrukturen, als auch flache Geflechte herstellbar. Ein flaches Geflecht kann anschließend in die Rohrform gebogen und an den sich berührenden Längskanten miteinander verbunden, beispielsweise verschweißt werden. Das Geflecht 11 kann sowohl in einer geschlossenen Struktur, als auch in einer offenen Struktur geflochten werden. Bei der geschlossenen Struktur wird der Flechtvorgang ausgehend von der Endmasche 18 gestartet. Dabei werden der erste Abschnitt 16a und der zweite Abschnitt 16b der Abschlusskante 16 gleichzeitig hergestellt. Alternativ kann die Gitterstruktur bzw. das Geflecht 11 als offene Struktur hergestellt werden. Das Geflecht 11 wird dabei kontinuierlich hergestellt und in gewünschte Längenabschnitte unterteilt, die jeweils offene Drahtenden aufweisen. Die offenen Drahtenden werden anschließend, beispielsweise per Hand, umgelenkt und verdrillt, um die Abschlusskante 16 zu bilden. Es ist auch möglich, dass die Verdrillung bereits in der Flechtmaschine erfolgt, so dass nach Teilen des kontinuierlichen Geflechtsstrangs die Abschlusskante 16 durch entsprechende Anordnung der verdrillten Abschnitte hergestellt wird. Dabei werden die verdrillten Abschnitte derart angeordnet, dass sich eine kontinuierlich glatte Abschlusskante 16 bildet.

Es ist auch möglich, die erfindungsgemäße Vorrichtung, insbesondere nach einem der vorstehend beschriebenen Ausführungsbeispiele, vollständig per Hand herzustellen.

### Bezugszeichenliste

- S: Schnittlinie
- L: Längsachse
- LR: Längsrichtung
- UR: Umfangsrichtung
- ULR: Umlaufrichtung

- 10: Hohlkörper
- 11: Geflecht
- 12: Drahtelement
- 12': inneres Drahtelement
- 12a: erstes äußeres Drahtelement
- 12b: zweites äußeres Drahtelement
- 12c: drittes äußeres Drahtelement
- 12d: viertes äußeres Drahtelement
- 12a': erstes inneres Drahtelement
- 12b': zweites inneres Drahtelement
- 12c': drittes inneres Drahtelement
- 12d': viertes inneres Drahtelement
- 12x: kantenbildendes Drahtelement
- 12x': geflechtbildendes Drahtelement
- 13: erste Reihe
- 14a: erste Endmasche
- 14b: zweite Endmasche
- 14c: dritte Endmasche
- 14d: vierte Endmasche
- 15: Geflechtende
- 15a: Rand
- 16: Abschlusskante
- 16a: erster Abschnitt
- 16b: zweiter Abschnitt
- 16c: vorderer Bereich
- 16d: hinterer Bereich
- 16e: Zwischenbereich
- 17b: erste Stelle
- 17c: zweite Stelle
- 17c: dritte Stelle
- 18: Endmasche
- 19: Verlängerung
- 20: Verbindungselement
- 21: Drahtbündel
- 22: distales Ende
- 23: "Klöppel
- 30: Zufuhrsystem
- 31: Katheter
- 32: Betätigungselement/Führungsdraht
- 33: Abkopplungsmechanismus
- 33a: proximales Ende
- 33b: Auslöseelement
- 34: Zwischenstück
- 35: Haltemittel
- 35a: erstes Arretierungselement

- 35b: zweites Arretierungselement
- 36: innere Maschengrenze
- 37a: erste Schlaufe
- 37b: zweite Schlaufe
- 37c: zweite Schlaufe
- 38: offene Zelle
- 39: Scheitel
- 40: Thrombus/Konkrement
- 50: Blutgefäß/Hohlorgan

## Patentansprüche

1. Medizinische Vorrichtung zur Einfuhr in ein Hohlorgan mit einem Hohlkörper (10), der ein Geflecht (11) aus Drahtelementen (12) mit einer Reihe (13) von Endmaschen (14a, 14b, 14c, 14d) aufweist, die ein axiales Geflechtende begrenzen, wobei die Endmaschen (14a, 14b, 14c, 14d) äußere Drahtelemente (12a, 12b, 12c, 12d) umfassen, die eine Abschlusskante (16) des Geflechts (11) bilden und in innere Drahtelemente (12a', 12b', 12c', 12d') übergehen, die innerhalb des Geflechts (11) angeordnet sind,
**dadurch gekennzeichnet, dass**
ein erster Abschnitt (16a) der Abschlusskante (16) und ein zweiter Abschnitt (16b) der Abschlusskante (16) jeweils mehrere äußere Drahtelemente (12a, 12b, 12c, 12d) aufweisen, die zusammen einen umlaufenden Rand (15a) der Abschlusskante (16) bilden, der angepasst ist derart, dass das axiale Geflechtende (15) des Hohlkörpers (10) in ein Zufuhrsystem einziehbar ist, wobei
- die äußeren Drahtelemente (12a, 12b, 12c, 12d) des ersten Abschnitts (16a) zur Bildung der Abschlusskante (16) entlang dieser einander unmittelbar nachgeordnet sind und jeweils eine erste Axialkomponente AK1 aufweisen, die in Längsrichtung L des Hohlkörpers (10) verläuft, und
- die äußeren Drahtelemente (12a, 12b, 12c, 12d) des zweiten Abschnitts (16b) zur Bildung der Abschlusskante (16) entlang dieser einander unmittelbar nachgeordnet sind und jeweils eine zweite Axialkomponente AK2 aufweisen, die in Längsrichtung L des Hohlkörpers (10) verläuft und der ersten Axialkomponente AK1 entgegengesetzt ist, wobei die beiden Axialkomponenten AK1, AK2 auf dieselbe Umlaufrichtung ULR des Randes (15a) bezogen sind.

2. Vorrichtung nach Anspruch 1,
**dadurch gekennzeichnet, dass**
die äußeren Drahtelemente (12a, 12b, 12c, 12d) des ersten Abschnitts (16a) eine erste Umfangskomponente UK1 aufweisen und die äußeren Drahtelemente (12a, 12b, 12c, 12d) des zweiten Abschnitts (16b) eine zweite Umfangskomponente UK2 aufweisen, wobei die erste und zweite Umfangskomponente UK1, UK2 in derselben Umfangsrichtung UR des Hohlkörpers (10) verlaufen, und/oder
die Abschlusskante (16) bezogen auf die Längsrichtung L des Hohlkörpers (10) schräg angeordnet ist.

3. Vorrichtung nach wenigstens einem der vorhergehenden Ansprüche,
**dadurch gekennzeichnet, dass**
sich wenigstens ein erstes äußeres Drahtelement (12a) einer Endmasche (14a) in den Bereich einer anderen Endmasche (14a) erstreckt, wobei sich das erste äußere Drahtelement (12a) und wenigstens ein zweites äußeres Drahtelement (12a, 12b, 12c, 12d) der anderen Endmasche (14b, 14c, 14d) überlappen und zusammen entlang der Abschlusskante (16) angeordnet sind, wobei insbesondere das wenigstens eine erste äußere Drahtelement (12a) und das wenigstens eine zweite äußere Drahtelement (12a, 12b, 12c, 12d) verbunden sind.

4. Vorrichtung nach wenigstens einem der vorhergehenden Ansprüche,
**dadurch gekennzeichnet, dass**
die Endmaschen (14a, 14b, 14c, 14d) durch Schlaufen gebildet sind, die entlang der Abschlusskante (16) versetzt angeordnet sind und sich überlappen, wobei die Schlaufen zur Bildung der miteinander verbundenen äußeren Drahtelemente (12a, 12b, 12c, 12d) an der Abschlusskante (16) an verschiedenen Stellen (17b, 17c, 17d) zusammengeführt sind, die entlang der Abschlusskante (16) einander nachgeordnet sind.

5. Vorrichtung nach wenigstens einem der vorhergehenden Ansprüche,
**dadurch gekennzeichnet, dass**
die im Geflecht (11) angeordneten inneren Drahtelemente (12a', 12b', 12c',
12d') der Abschlusskante (16) zugeführt oder von der Abschlusskante (16) abgezweigt sind.

6. Vorrichtung nach Anspruch 5,
**dadurch gekennzeichnet, dass**
die inneren Drahtelemente (12a', 12b', 12c', 12d') beim Übergang in die äußeren Drahtelemente (12a, 12b, 12c, 12d) die Richtung der Umfangskomponente bezogen auf die Umfangsrichtung und/oder die Richtung der Axialkomponente bezogen auf die Längsrichtung L des Hohlkörpers (10) ändern, wobei insbesondere die inneren Drahtelemente (12a', 12b', 12c', 12d') und äußeren Drahtelemente (12a, 12b, 12c, 12d) denselben Flechtwinkel aufweisen.

7. Vorrichtung nach wenigstens einem der vorhergehenden Ansprüche,
**dadurch gekennzeichnet, dass**
die miteinander verbundenen äußeren Drahtelemente (12a, 12b, 12c, 12d) der Endmaschen (14a, 14b, 14c, 14d) formschlüssig, insbesondere durch Verdrillen oder Verflechten, oder stoffschlüssig, insbesondere durch Verkleben, Verschweißen oder Verlöten, oder durch mechanische Verbindungsmittel, insbesondere durch Coils und/oder Hülsen und/oder Drähte, verbunden sind.

8. Vorrichtung nach wenigstens einem der vorhergehenden Ansprüche,
**dadurch gekennzeichnet, dass**
die Anzahl der äußeren Drahtelemente (12a, 12b, 12c, 12d) des ersten Abschnitts (16a) und des zweiten Abschnitts (16b) jeweils zu einem in Längsrichtung L des Hohlkörpers (10) vorderen Bereich (16c) der Abschlusskante (16) zunimmt und im vorderen Bereich (16c) maximal ist, wobei insbesondere die äußeren Drahtelemente (12a, 12b, 12c, 12d) der Endmaschen (14a, 14b, 14c, 14d) in einer vorderen Endmasche (18) zusammengeführt sind.

9. Vorrichtung nach Anspruch 8,
**dadurch gekennzeichnet, dass**
die äußeren Drahtelemente (12a, 12b, 12c, 12d) eine einzige Gruppe bilden, die im Bereich der vorderen Endmasche (18) umgelenkt ist, insbesondere die äußeren Drahtelemente (12a, 12b, 12c, 12d) wenigstens zwei Gruppen bilden, die im Bereich der vorderen Endmasche (18) verbunden sind.

10. Vorrichtung nach wenigstens einem der Ansprüche 1 bis 7,
**dadurch gekennzeichnet, dass**
die Anzahl der äußeren Drahtelemente (12a, 12b, 12c, 12d) des ersten Abschnitts (16a) und des zweiten Abschnitts (16b) jeweils zu einem in Längsrichtung L des Hohlkörpers (10) vorderen Bereich (16c) der Abschlusskante (16) hin abnimmt und im vorderen Bereich (16c) minimal ist, wobei insbesondere die vom vorderen Bereich der Abschlusskante (16) entfernt angeordneten äußeren Drahtelemente (12a, 12b, 12c, 12d) des ersten Abschnitts (16a) und/oder des zweiten Abschnitts (16b) jeweils ein Drahtbündel (21) bilden, das zur Verstärkung des Hohlkörpers (10) im Geflecht (11) angeordnet ist.

11. Vorrichtung nach wenigstens einem der Ansprüche 1 bis 8,
**dadurch gekennzeichnet, dass**
zwischen einem vorderen Bereich (16c) und einem hinteren Bereich (16d) der Abschlusskante (16) ein Zwischenbereich (16e) angeordnet ist und die Anzahl der äußeren Drahtelemente (12a, 12b, 12c, 12d) ausgehend vom Zwischenbereich (16e) zum vorderen Bereich (16c) und zum hinteren Bereich (16d) der Abschlusskante (16) hin jeweils abnimmt, wobei die Anzahl der äußeren Drahtelemente (12a, 12b, 12c, 12d) im Zwischenbereich (16e) maximal ist.

12. Vorrichtung nach Anspruch 1 oder 2,
**dadurch gekennzeichnet, dass**
die inneren Drahtelemente (12a', 12b', 12c', 12d') innere Maschengrenzen (36a, 36b) der Endmaschen (14a, 14b, 14c, 14d) des ersten Abschnitts (16a) und/oder des zweiten Abschnitts (16b) bilden, die sich ausgehend von der Abschlusskante (16) ins Geflechtinnere erstrecken, wobei die Endmaschen (14a, 14b, 14c, 14d) zumindest teilweise an den inneren Maschengrenzen (36, 36b) miteinander verbunden sind, wobei insbesondere die äußeren Drahtelemente (12a, 12b, 12c, 12d) der Endmaschen (14a, 14b, 14c, 14d) nicht überlappen.

13. Vorrichtung nach Anspruch 1 oder 2,
**dadurch gekennzeichnet, dass**
die Endmaschen (14a, 14b, 14c, 14d) des ersten Abschnitts (16a) und/oder des zweiten Abschnitts (16b) jeweils wenigstens zwei Schlaufen (37a, 37b) aufweisen, die entlang der Abschlusskante (16) nebeneinander angeordnet sind, wobei wenigstens eine dritte Schlaufe (37c) die erste Schlaufe (37a) und zweite Schlaufe (37b) überlappt.

14. Vorrichtung nach wenigstens einem der vorhergehenden Ansprüche,
**dadurch gekennzeichnet, dass**
wenigstens ein äußeres Drahtelement (12a, 12b, 12c, 12d) und/oder ein zusätzliches Drahtelement eine Verlängerung (19) bildet, die sich über die Kontur der Abschlusskante (16) hinaus erstreckt.

15. Verfahren zur Herstellung einer medizinischen Vorrichtung gemäß Anspruch 1, bei dem ein Hohlkörper (10) aus Drahtelementen geflochten und eine Reihe von nebeneinander angeordneten Endmaschen (14a, 14b, 14c, 14d) gebildet wird, die ein axiales Geflechtende begrenzen, wobei jede Endmasche (14a, 14b, 14c, 14d) wenigstens ein äußeres Drahtelement (12b, 12c, 12d) umfasst und die äußeren Drahtelemente (12a, 12b, 12c, 12d) der Endmaschen (14a, 14b, 14c, 14d) zusammen eine Abschlusskante (16) des Geflechtendes (11) bilden, wobei im Geflecht (11) angeordnete innere Drahtelemente (12a', 12b', 12c', 12d') zur Bildung der äußeren Drahtelemente (12a, 12b, 12c, 12d) umgelenkt werden derart, dass die Windungsrichtung und/oder die Richtung der Axialkomponente bezogen auf die Längsachse L der inneren Drahtelemente (12a', 12b', 12c', 12d') geändert wird.

## Claims

1. A medical device for insertion into a hollow organ with a hollow body (10), which comprises a braid (11) made of wire elements (12) with a series (13) of terminal meshes (14a, 14b, 14c, 14d), which delimit an axial braid end, wherein the terminal meshes (14a, 14b, 14c, 14d) comprise external wire elements (12a, 12b, 12c, 12d) which form a closing edge (16) of the braid (11) and make a transition to internal wire elements (12a', 12b', 12c', 12d') which are arranged inside the braid (11),
**characterised in that**
a first section (16a) of the closing edge (16) and a second section (16b) of the closing edge (16) each comprise a plurality of external wire elements (12a, 12b, 12c, 12d), which together form a circumferential rim (15a) of the closing edge (16), which is adapted in such a way that the axial braid end (15) of the hollow body (10) can be drawn into a feeding system, wherein
- the external wire elements (12a, 12b, 12c, 12d) of the first section (16a), in order to form the closing edge (16), are arranged along the same directly one after the other and comprise a first axial component AK1 each, which extends in longitudinal direction L of the hollow body (10), and
- the external wire elements (12a, 12b, 12c, 12d) of the second section (16b), in order to form the closing edge (16), are arranged along the same directly one after the other and comprise a second axial component AK2 each, which extends in longitudinal direction L of the hollow body (10), and in the opposite direction to the first axial component AK1, wherein both axial components AK1, AK2 are related to the same circulating direction ULR of the rim (15a).

2. The device according to claim 1,
**characterised in that**
the external wire elements (12a, 12b, 12c, 12d) of the first section (16a) have a first circumferential component UK1 and the external wire elements (12a, 12b, 12c, 12d) of the second section (16b) have a second circumferential component UK2, wherein the first and second circumferential components UK1, UK2, extend in the same circumferential direction UR of the hollow body (10),
and/or
the closing edge (16), in relation to the longitudinal direction L of the hollow body (10), is arranged obliquely.

3. The device according to at least one of the preceding claims,
**characterised in that**
at least one first external wire element (12a) of a terminal mesh (14a) extends into the area of another terminal mesh (14a), wherein the first external wire element (12a) and at least one second external wire element (12a, 12b, 12c, 12d) of the other terminal mesh (14b, 14c, 14d) overlap and are arranged both along the closing edge (16), wherein in particular the at least one first external wire element (12a) and the at least one second external wire element (12a, 12b, 12c, 12d) are connected.

4. The device according to at least one of the preceding claims,
**characterised in that**
the terminal meshes (14a, 14b, 14c, 14d) are formed of loops which are arranged in a staggered fashion along the closing edge (16) and which overlap each other, wherein in order to form the interconnected external wire elements (12a, 12b, 12c, 12d) on the closing edge (16), the loops are joined together at various places (17b, 17c, 17d) arranged one after the other along the closing edge (16).

5. The device according to at least one of the preceding claims,
**characterised in that**
the internal wire elements (12a', 12b', 12c', 12d') arranged in the braid (11) lead towards the closing edge (16) or branch out of the closing edge (16).

6. The device according to claim 5,
**characterised in that**
the internal wire elements (12a', 12b', 12c', 12d'), where they change into external wire elements (12a, 12b, 12c, 12d), change the direction of the circumferential component in relation to the circumferential direction and/or the direction of the axial component in relation to the longitudinal direction L of the hollow body (10), wherein in particular the internal wire elements (12a', 12b', 12c', 12d') and the external wire elements (12a, 12b, 12c, 12d) have the same braiding angle.

7. The device according to at least one of the preceding claims,
**characterised in that**,
the interconnected external wire elements (12a, 12b, 12c, 12d) of the terminal meshes (14a, 14b, 14c, 14d) are positively connected, in particular by twisting or braiding, or bonded together, in particular by gluing, welding or soldering, or by using mechanical connecting means, in particular coils and/or sleeves and/or wires.

8. The device according to at least one of the preceding claims,
**characterised in that**
the number of external wire elements (12a, 12b, 12c, 12d) in the first section (16a) and the second section (16b) respectively increases in a front area (16c) of the closing edge (16) viewed in longitudinal direction L of the hollow body (10) and reaches a maximum in the front area (16c), wherein in particular the external wire elements (12, 12b, 12c, 12d) of the terminal meshes (14a, 14b, 14c, 14d) are united in a front terminal mesh (18).

9. The device according to claim 8,
**characterised in that**
the external wire elements (12a, 12b, 12c, 12d) form a single group, which is deflected in the area of the front terminal mesh (18), in particular **in that** the external wire elements (12a, 12b, 12c, 12d) form at least two groups which are connected in the area of the front terminal mesh (18).

10. The device according to one of claims 1 to 7,
**characterised in that**
the number of external wire elements (12a, 12b, 12c, 12d) in the first section (16a) and the second section (16b) respectively decreases in the front area (16c) of the closing edge (16) viewed in longitudinal direction L of the hollow body (10) and reaches a minimum in the front area (16c), wherein in particular the external wire elements (12a, 12b, 12c, 12d) of the first section (16a) and/or the second section (16b) which are located remotely from the front area of the closing edge (16) each form a wire bundle (21), which is disposed in the braid (11) so as to strengthen the hollow body (10).

11. The device according to one of claims 1 to 8,
**characterised in that**
an intermediate area (16e) is arranged between a front area (16c) and a back area (16d) of the closing edge (16) and **in that** the number of external wire elements (12a, 12b, 12c, 12d) going out from the intermediate area (16e) to the front area (16c) and to the back area (16d) of the closing edge (16), respectively, decreases, wherein the number of external wire elements (12a, 12b, 12c, 12d) in the intermediate area (16e) is at a maximum.

12. The device according to one of claims 1 or 2,
**characterised in that**
the internal wire elements (12a', 12b', 12c', 12d') form inner mesh limits (36a, 36b) of the terminal meshes (14a, 14b, 14c, 14d) of the first section (16a) and/or of the second section (16b), which extend from the closing edge into the interior of the braid, wherein the terminal meshes (14a, 14b, 14c, 14d) are at least partially connected with each other at the inner mesh limits (36, 36b), wherein in particular the external wire elements (12a, 12b, 12c, 12d) of the terminal meshes (14a, 14b, 14c, 14d) do not overlap.

13. The device according to one of claims 1 or 2,
**characterised in that**,
the terminal meshes (14a, 14b, 14c, 14d) of the first section (16a) and/or the second section (16b) comprise at least two loops (37a, 37b) each, which are arranged adjacent to each other along the closing edge (16), wherein at least one third loop (37c) overlaps the first loop (37a) and the second loop (37b).

14. The device according to at least one of the preceding claims,
**characterised in that**,
at least one external wire element (12a, 12b, 12c, 12d) and/or one additional wire element forms an extension (19) which extends beyond the contour of the closing edge (16).

15. A method for producing a medical device according to claim 1, in which a hollow body (10) is braided from wire elements and a series of adjacently arranged terminal meshes (14a, 14b, 14c, 14d) is formed, which delimit an axial braid end, wherein each terminal mesh (14a, 14b, 14c, 14d) comprises at least one external wire element (12b, 12c, 12d), and the external wire elements (12a, 12b, 12c, 12d) of the terminal meshes (14a, 14b, 14c, 14d) together form a closing edge (16) of the braid end (11), wherein internal wire elements (12a', 12b', 12c', 12d') arranged inside the braid (11) are deflected to form the external wire elements (12a, 12b, 12c, 12d) such that the winding direction and/or the direction of the axial component is changed relative to the longitudinal axis L of the internal wire elements (12a', 12b', 12c', 12d').

## Revendications

1. Dispositif médical pour l'introduction dans un organe creux avec un corps creux (10), lequel présente un treillis (11) en éléments de fil (12) avec une rangée (13) de mailles d'extrémité (14a, 14b, 14c, 14d) qui délimitent une extrémité de treillis axiale, les mailles d'extrémité (14a, 14b, 14c, 14d) comprenant des éléments de fil extérieurs (12a, 12b, 12c, 12d) qui forment une arête de terminaison (16) du treillis (11) et font la transition éléments de fil intérieurs (12a', 12b', 12c', 12d') qui sont disposés à l'intérieur du treillis (11),
**caractérisé en ce**
**qu'**une première partie (16a) de l'arête de terminaison (16) et une deuxième partie (16b) de l'arête de terminaison (16) présentent respectivement plusieurs éléments de fil extérieurs (12a, 12b, 12c, 12d) formant ensemble un bord périphérique (15a) de l'arête de terminaison (16), lequel est adapté de manière à ce que l'extrémité de treillis axiale (15) du corps creux (10) puisse être rentrée dans un système d'alimentation, dans lequel
- pour la formation de l'arête de terminaison (16), les éléments de fil extérieurs (12a, 12b, 12c, 12d) de la première partie (16a) sont disposés le long de l'arête, directement en aval les uns des autres, et présentent respectivement une première composante axiale AK1 s'étendant en direction longitudinale L du corps creux (10), et
- pour la formation de l'arête de terminaison (16), les éléments de fil extérieurs (12a, 12b, 12c, 12d) de la deuxième partie (16b) étant disposés le long de l'arête, directement en aval les uns des autres, et présentant respectivement une deuxième composante axiale AK2 qui s'étend en direction longitudinale L du corps creux (10) et qui est opposée à la première composante axiale AK1, les deux composantes axiales AK1, AK2 étant en rapport avec la même direction périphérique ULR du bord (15a).

2. Dispositif selon la revendication 1,
**caractérisé en ce que**
les éléments de fil extérieurs (12a, 12b, 12c, 12d) de la première partie (16a) présentent une première composante circonférentielle UK1 et les éléments de fil extérieurs (12a, 12b, 12c, 12d) de la deuxième partie (16b), une deuxième composante circonférentielle UK2, la première et la deuxième composante circonférentielle UK1, UK2 s'étendant dans la même direction circonférentielle UR du corps creux (10),
et/ou
l'arête de terminaison (16) étant disposée en biais compte tenu de la direction longitudinale L du corps creux (10).

3. Dispositif selon l'une au moins des revendications précédentes,
**caractérisé en ce**
**qu'**au moins un premier élément de fil extérieur (12a) d'une maille d'extrémité (14a) s'étend dans la zone d'une autre maille d'extrémité (14a), le premier élément de fil extérieur (12a) et au moins un deuxième élément de fil extérieur (12a, 12b, 12c, 12d) de l'autre maille d'extrémité (14b, 14c, 14d) se chevauchant et étant disposés ensemble le long de l'arête de terminaison (16), cet au moins un premier élément de fil extérieur (12a) et cet au moins un deuxième élément de fil extérieur (12a, 12b, 12c, 12d) étant en particulier reliés.

4. Dispositif selon l'une au moins des revendications précédentes,
**caractérisé en ce que**
les mailles d'extrémité (14a, 14b, 14c, 14d) sont formées par des boucles qui sont disposées de manière décalée le long de l'arête de terminaison (16) et se chevauchent, les boucles étant réunies, pour la formation des éléments de fil extérieurs (12a, 12b, 12c, 12d) reliés ensemble au niveau de l'arête de terminaison (16), à divers endroits (17b, 17c, 17d), lesquels sont disposés directement en aval les uns des autres le long de l'arête de terminaison (16).

5. Dispositif selon l'une au moins des revendications précédentes,
**caractérisé en ce que**
les éléments de fil intérieurs (12a', 12b', 12c', 12d') disposés dans le treillis (11) sont menés vers l'arête de terminaison (16) ou bien bifurquent à partir de l'arête de terminaison (16).

6. Dispositif selon la revendication 5,
**caractérisé en ce que**
les éléments de fil intérieurs (12a', 12b', 12c', 12d'), lors de la transition pour devenir les éléments de fil extérieurs (12a, 12b, 12c, 12d), modifient la direction de la composante circonférentielle compte tenu de la direction circonférentielle et/ou la direction de la composante axiale compte tenu de la direction longitudinale L du corps creux (10), les éléments de fil intérieurs (12a', 12b', 12c', 12d') et les éléments de fil extérieurs (12a, 12b, 12c, 12d) présentant en particulier le même angle de tressage.

7. Dispositif selon l'une au moins des revendications précédentes,
**caractérisé en ce que**
les éléments de fil extérieurs (12a, 12b, 12c, 12d), reliés entre eux, des mailles d'extrémité (14a, 14b, 14c, 14d), sont reliés par conjugaison de formes, en particulier par torsadage ou entrelacement, ou par conjugaison de matières, en particulier par collage, soudage ou brasage, ou bien grâce à des moyens de liaison mécaniques, en particulier grâce à des bobines et/ou manchons et/ou fils.

8. Dispositif selon l'une au moins des revendications précédentes,
**caractérisé en ce que**
le nombre d'éléments de fil extérieurs (12a, 12b, 12c, 12d) de la première partie (16a) et de la deuxième partie (16b) augmente respectivement vers une zone antérieure (16c), en direction longitudinale L du corps creux (10), de l'arête de terminaison (16) et est maximal dans la zone antérieure (16c), les éléments de fil extérieurs (12a, 12b, 12c, 12d) des mailles d'extrémité (14a, 14b, 14c, 14d) étant en particulier réunis dans une maille d'extrémité antérieure (18).

9. Dispositif selon la revendication 8,
**caractérisé en ce que**
les éléments de fil extérieurs (12a, 12b, 12c, 12d) forment un seul groupe qui est dévié dans la zone de la maille d'extrémité antérieure (18), les éléments de fil extérieurs (12a, 12b, 12c, 12d) formant en particulier au moins deux groupes qui sont reliés dans la zone de la maille d'extrémité antérieure (18).

10. Dispositif selon l'une au moins des revendications 1 à 7,
**caractérisé en ce que**
le nombre d'éléments de fil extérieurs (12a, 12b, 12c, 12d) de la première partie (16a) et de la deuxième partie (16b) diminue respectivement vers une zone antérieure (16c), en direction longitudinale L du corps creux (10), de l'arête de terminaison (16) et est minimal dans la zone antérieure (16c), les éléments de fil extérieurs (12a, 12b, 12c, 12d), disposés de manière éloignée de la zone antérieure de l'arête de terminaison (16), de la première partie (16a) et/ou de la deuxième partie (16b) formant en particulier respectivement un faisceau de fils (21) qui est disposé dans le treillis (11) pour le renforcement du corps creux (10).

11. Dispositif selon l'une au moins des revendications 1 à 8,
**caractérisé en ce**
**qu'**une zone intermédiaire (16e) est disposée entre la zone antérieure (16c) et une zone postérieures (16d) de l'arête de terminaison (16), et en ce que le nombre d'éléments de fil extérieurs (12a, 12b, 12c, 12d) diminue, en partant de la zone intermédiaire (16e), respectivement vers la zone antérieure (16c) et la zone postérieure (16d) de l'arête de terminaison (16), le nombre d'éléments de fil extérieurs (12a, 12b, 12c, 12d) étant maximal dans la zone intermédiaire (16e).

12. Dispositif selon la revendication 1 ou 2,
**caractérisé en ce que**
les éléments de fil intérieurs (12a', 12b', 12c', 12d') forment des limites de mailles intérieures (36a, 36b) des mailles d'extrémité (14a, 14b, 14c, 14d) de la première partie (16a) et/ou de la deuxième partie (16b), lesquelles s'étendent à l'intérieur du treillis en partant de l'arête de terminaison (16), les mailles d'extrémité (14a, 14b, 14c, 14d) étant au moins partiellement reliées entre elles au niveau des limites de maille intérieures (36, 36b), les éléments de fil extérieurs (12a, 12b, 12c, 12d) des mailles d'extrémité (14a, 14b, 14c, 14d) ne se chevauchant en particulier pas.

13. Dispositif selon la revendication 1 ou 2,
**caractérisé en ce que**
les mailles d'extrémité (14a, 14b, 14c, 14d) de la première partie (16a) et/ou de la deuxième partie (16b) présentent respectivement au moins deux boucles (37a, 37b), lesquelles sont disposées côte à côte le long de l'arête de terminaison (16), au moins une troisième boucle (37c) chevauchant la première boucle (37a) et la deuxième boucle (37b).

14. Dispositif selon l'une au moins des revendications précédentes,
**caractérisé en ce**
**qu'**au moins un élément de fil extérieur (12a, 12b, 12c, 12d) et/ou un élément de fil supplémentaire forme une prolongation (19) qui s'étend au-delà du contour de l'arête de terminaison (16).

15. Procédé pour la fabrication d'un dispositif médical selon la revendication 1, dans lequel un corps creux (10) est tressé à partir d'éléments de fil, et une rangée de mailles d'extrémité (14a, 14b, 14c, 14d) disposées les unes à côté des autres étant formée, lesquelles délimitent une extrémité de treillis axiale, chaque maille d'extrémité (14a, 14b, 14c, 14d) comprenant au moins un élément de fil extérieur (12b, 12c, 12d), et les éléments de fil extérieurs (12a, 12b, 12c, 12d) des mailles d'extrémité (14a, 14b, 14c, 14d) formant ensemble une arête de terminaison (16) de l'extrémité de treillis (11), des éléments de fil intérieurs (12a', 12b', 12c', 12d') disposés à l'intérieur du treillis (11) étant déviés pour la formation des éléments de fils extérieurs (12a, 12b, 12c, 12d) de manière à ce que la direction de détour et/ou la direction de la composante axiale soit modifiée en fonction de l'axe longitudinal L des éléments de fil intérieurs (12a', 12b', 12c', 12d').
